# EUROPÄISCHE PATENTSCHRIFT

(11) **EP 3 463 271 B1**
(45) Veröffentlichungstag und Bekanntmachung des Hinweises auf die Patenterteilung: **27.07.2022**
(21) Anmeldenummer: 17726642.6
(22) Anmeldetag: 31.05.2017
(51) Int. Cl.: A61K 8/362, A61K 8/44, A61K 8/49, A61Q 5/06, A61Q 5/08, A61Q 5/10, A61K 8/22, A61K 8/34, A61K 8/365, A61K 8/41

(54) **SCHONENDE MITTEL UND VERFAHREN ZUR OXIDATIVEN FÄRBUNG MIT AUSGEWÄHLTEN DICARBONSÄUREN**
GENTLE AGENTS AND METHODS FOR OXIDATIVE DYEING USING SELECTED DICARBOXYLIC ACIDS
PRODUIT ET PROCÉDÉ DE COLORATION D'OXYDATION DOUX FAISANT APPEL À DES ACIDES DICARBOXYLIQUES SÉLECTIONNÉS

(30) Priorität: 31.05.2016 DE 102016209468
(43) Veröffentlichungstag der Anmeldung: 10.04.2019
(73) Patentinhaber: Henkel AG & Co. KGaA, 40589 Düsseldorf (DE)
(72) Erfinder: MANNECK, Hartmut, 23858 Barnitz (DE); HIPPE, Thomas, 25482 Appen (DE); KLEEN-FEHRES, Astrid, 20457 Hamburg (DE)
(86) Internationale Anmeldenummer: PCT/EP2017/063161
(87) Internationale Veröffentlichungsnummer: WO 2017/207631

(56) Entgegenhaltungen:
- EP-A1- 0 765 154
- WO-A1-2005/115314
- WO-A1-2017/085117
- DE-A1- 10 051 774
- DE-A1-102015 222 946
- GB-A- 1 136 659
- GB-A- 2 259 717
- US-A1- 2011 247 644
- US-B1- 6 515 114

## Beschreibung

Die vorliegende Erfindung betrifft ein das Haar schonendes Mittel zur oxidativen Haarfärbung sowie ein schonendes Verfahren zur oxidativen Haarfärbung, bei dem keratinische Fasern vor oxidativen Einflüssen geschützt bzw. oxidative Haarschäden repariert werden.

Beim oxidativen Färben von Haaren kann es aufgrund der aggressiven Agentien zu Schäden an der keratinischen Faser kommen. Insbesondere die natürliche Hydrophobizität der keratinischen Faser wird reduziert, da die Färbemittel das Haar zunächst penetrationsfähig machen müssen, um ihre Wirkung zu entfalten. Die wasserabweisende Wirkung ist aber einerseits ein natürlicher Schutz des Haares, andererseits sind vom Verbraucher erwünschte Parameter wie Glanz, Geschmeidigkeit, Griff und "Fallen" der Haare eng mit ihr verknüpft.

Um die genannten Nachteile zu überwinden, sind so genannte Vorbehandlungsmittel auf dem Markt, die das Haar vor dem aggressiven Einfluss schützen sollen. Diese beschweren das Haar aber oft oder beeinträchtigen den Erfolg der im Anschluss stattfindenden Aufhellung bzw. Färbung des Haares. Insbesondere die Waschechtheit der Färbung kann durch das Vorbehandlungsmittel verschlechtert sein. Auch sind zahlreiche Nachbehandlungsmittel bekannt, mit denen versucht wird, die bei der oxidativen Färbebehandlung verursachten Haarschädigungen zu reparieren. Alle diese Verfahren erfordern aber ein mehrstufiges Anwendungsverfahren, eben entweder eine dem Färben vor- oder nachgelagerte Applikation eines weiteren Haarbehandlungsmittels. Dies wird vom Verbraucher häufig als lästig wahrgenommen, da bereits die oxidative Färbebehandlung selbst mit mehreren Arbeitsschritten und einer Einwirkzeit von bis zu 60 Minuten sehr aufwendig ist.

Der vorliegenden Erfindung lag die Aufgabe zugrunde, ein Mittel und ein Verfahren zur oxidativen Haarfärbung mit einer Haar schützenden Behandlung bereitzustellen, das die genannten Nachteile überwindet, ohne das Farbergebnis der oxidativen Färbebehandlung negativ zu beeinflussen. Dabei sollten insbesondere ein Färbemittel und ein Verfahren bereitgestellt werden, bei dem das Haar nicht beschwert wird und eine möglichst geringe Haarschädigung auftritt. Weiterhin sollte der erzielte Haarschutz möglichst wenig zeitaufwendig sein und möglichst zusammen mit dem Färbeschritt selbst erfolgen.

Der Einsatz von Dicarbonsäuren wie Bernsteinsäure in der Haarpflege ist Stand der Technik. Diese werden in Shampoos und insbesondere in Conditionern breit eingesetzt, um dort Pflegeeffekte zu entfalten. So offenbart die Patentanmeldung WO 2005/115314A1 ein Verfahren zur Restrukturierung keratinischer Fasern, bei dem die Keratinfasern mit Cystin und mit mindestens einer Dicarbonsäure mit 2 bis 10 Kohlenstoffatomen in Kontakt gebracht werden, wobei bevorzugte Dicarbonsäuren ausgewählt sind aus Oxalsäure, Malonsäure, Bernsteinsäure, Glutarsäure, Adipinsäure, Pimelinsäure, Azelainsäure, Maleinsäure, Fumarsäure und Sorbinsäure und Bernsteinsäure besonders bevorzugt ist. Die Patentanmeldung DE 10051774 A1 beschreibt die Verwendung kurzkettiger Carbonsäuren mit einem Molekulargewicht unter 750 g/mol in kosmetischen Mitteln als Wirkstoff zur Restrukturierung keratinischer Fasern. Die Patentanmeldung EP1174112A offenbart Haarbehandlungsmittel, die neben einer organischen Säure als weitere zwingende Bestandteile ein organisches Lösungsmittel, ein kationisches Tensid und einen höheren Alkohol enthalten und zur Reparatur von Poren in Haaren dienen.

In neuerer Zeit werden im Markt auch Agentien angeboten, die Färbezusammensetzungen zum Zwecke des Faserschutzes zugemischt werden sollen und Dicarbonsäuren enthalten. Bei den genannten Mitteln erfolgt zwar keine dem Färben vor- oder nachgelagerte Applikation eines weiteren Haarbehandlungsmittels, doch muss dieses Mittel vor der Applikation mit dem eigentlichen Färbemittel vermischt werden, was ebenfalls einen weiteren Arbeitsschritt bedeutet und vom Verbraucher als lästig empfunden wird.

Es wurde nun gefunden, dass oxidative Färbemittel mit verbessertem Faserschutz bereitgestellt werden können, wenn die im Verlaufe der Vorbereitung durch den Kunden mit dem Entwickler zu vermischende Färbecreme neben typischen Bestandteilen wie Wasser und Farbstoffen bzw. deren Vorprodukten mindestens eine Dicarbonsäure mit 2 bis 10 Kohlenstoffatomen enthält und weitgehend frei ist von Peroxiden. Dabei wird nicht nur ein weiterer Arbeitsschritt vermieden, vielmehr sind diese Mittel bei ansonsten gleichen Einsatzmengen der Dicarbonsäure(n) auch gegenüber der nachträglichen Zumischung effektiver im Faserschutz

Gegenstand der vorliegenden Erfindung sind in einer ersten Ausführungsform Färbemittel für keratinische Fasern, insbesondere für Humanhaar, enthaltend - bezogen auf ihr Gewicht -
a) mindestens eine Verbindung, ausgewählt aus der Gruppe von Oxidationsfarbstoffvorprodukten, direktziehenden Farbstoffen sowie deren Mischungen,
b) 0,1 bis 5 Gew.-%, jeweils umgerechnet auf die undissoziierte Dicarbonsäure und bezogen auf das Gewicht des Färbemittels, Dicarbonsäure(n) mit 2 bis 10 Kohlenstoffatomen, ausgewählt aus Äpfelsäure, D-Weinsäure, L-Weinsäure, meso-Weinsäure, Traubensäure, alpha-Ketoglutarsäure, beta-Ketoglutarsäure und Oxalessigsäure, und/oder Salz(e) dieser Säure(n),
c) 20 bis 95 Gew.-% Wasser und
d) weniger als 0,1 Gew.-% Peroxidverbindung(en),
   dadurch gekennzeichnet, dass 0,05 bis 5 Gew.-%, vorzugsweise 0,1 bis 4,5 Gew.-%, weiter bevorzugt 0,15 bis 4 Gew.-%, noch weiter bevorzugt 0,2 bis 3,5 Gew.-% und insbesondere 0,25 bis 3 Gew.-% Oxidationsfarbstoffvorprodukte enthalten sind,
   weiterhin dadurch gekennzeichnet, dass die mindestens eine Dicarbonsäure mit 2 bis 10 Kohlenstoffatomen ausgewählt ist aus Äpfelsäure,
   weiterhin dadurch gekennzeichnet, dass es zusätzlich Mischungen aus Arginin und Lysin in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Aminosäure und bezogen auf das Gewicht des Färbemittels, enthält, wobei
   mindestens eine Aminosäure aus der Gruppe Arginin, Lysin, Histidin, Asparagin, Glutamin, Cystein, Methionin, Tryptophan sowie Mischungen hiervon in einer Gesamtmenge von 0,05 bis 3 Gew.-%, bevorzugt 0,1 bis 2 Gew.-%, besonders bevorzugt 0,2 bis 1,2 Gew.-%, enthalten ist, jeweils umgerechnet auf die undissoziierte Aminosäure und bezogen auf das Gewicht des Färbemittels, und
   gekennzeichnet durch einen pH-Wert im Bereich von 8,5 bis 11,0, bevorzugt 10 bis 10,5, jeweils gemessen bei 20°C.

Ein weiterer Gegenstand der vorliegenden Erfindung ist ein Verfahren zur oxidativen Färbung keratinischer Fasern, insbesondere von Humanhaar, das folgende Verfahrensschritte umfasst
I. Bereitstellen einer Zusammensetzung (A), enthaltend - bezogen auf ihr Gewicht -
   a) mindestens eine Verbindung, ausgewählt aus der Gruppe von Oxidationsfarbstoffvorprodukten, direktziehenden Farbstoffen sowie deren Mischungen,
   b) 0,1 bis 5 Gew.-%, jeweils umgerechnet auf die undissoziierte Dicarbonsäure und bezogen auf das Gewicht der Zusammensetzung (A), Dicarbonsäure(n) mit 2 bis 10 Kohlenstoffatomen, ausgewählt aus Äpfelsäure, D-Weinsäure, L-Weinsäure, meso-Weinsäure, Traubensäure, alpha-Ketoglutarsäure, beta-Ketoglutarsäure und Oxalessigsäure, und/oder Salz(e) dieser Säure(n),
   c) 20 bis 95 Gew.-% Wasser und
   d) weniger als 0,1 Gew.-% Peroxidverbindung(en),
   e) 0,05 bis 5 Gew.-%, vorzugsweise 0,1 bis 4,5 Gew.-%, weiter bevorzugt 0,15 bis 4 Gew.-%, noch weiter bevorzugt 0,2 bis 3,5 Gew.-% und insbesondere 0,25 bis 3 Gew.-% Oxidationsfarbstoffvorprodukte,
   f) Mischungen aus Arginin und Lysin, in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Aminosäure und bezogen auf das Gewicht des Färbemittels, wobei
      mindestens eine Aminosäure aus der Gruppe Arginin, Lysin, Histidin, Asparagin, Glutamin, Cystein, Methionin, Tryptophan sowie Mischungen hiervon in einer Gesamtmenge von 0,05 bis 3 Gew.-%, bevorzugt 0,1 bis 2 Gew.-%, besonders bevorzugt 0,2 bis 1,2 Gew.-%, enthalten ist, jeweils umgerechnet auf die undissoziierte Aminosäure und bezogen auf das Gewicht des Färbemittels,
      dadurch gekennzeichnet, dass die mindestens eine Dicarbonsäure mit 2 bis 10 Kohlenstoffatomen ausgewählt ist aus Äpfelsäure und
      gekennzeichnet durch einen pH-Wert im Bereich von 8,5 bis 11,0, bevorzugt 10 bis 10,5, jeweils gemessen bei 20°C,
II. Bereitstellen einer Zusammensetzung (B), enthaltend mindestens eine Peroxidverbindung, die bevorzugt Wasserstoffperoxid ist,
   wobei die Zusammensetzung (B) bevorzugt einen pH-Wert im Bereich von 2,5 bis 6,5, bevorzugt 3,0 - 5,5, besonders bevorzugt 3,5 bis 5,0, aufweist, jeweils gemessen bei 20°C,
III. Vermischen der Zusammensetzungen (A) und (B) miteinander, direkt anschließend
IV. Auftragen der Mischung aus (A) und (B) auf die keratinischen Fasern, insbesondere auf das Humanhaar, und
V. Ausspülen nach einer Einwirkzeit von 0,1 bis 60 Minuten, bevorzugt 1 bis 45 Minuten, besonders bevorzugt 10 bis 30 Minuten,
VI. ggf. weitere Haarbehandlungen, wie Verformen, Konditionieren und/oder Trocknen.

Die erfindungsgemäßen bzw. im erfindungsgemäßen Verfahren eingesetzten Mittel enthalten bevorzugt mindestens eine Kupplerkomponente. Besonders schöne Färbungen können erzielt werden, wenn als Kupplerkomponente mindestens eine Kupplerkomponente in den Mitteln enthalten ist, die ausgewählt ist aus der Gruppe die gebildet wird aus 3-Amino-2-methylamino-6-meth-oxypyridin, 3-Amino-6-methylphenol, 3-Amino-2-hydroxypyridin, 1,3-Bis-(2,4-diaminophenoxy)pro-pan, 2,7-Dihydroxynaphthalin, 2-Methylresorcin, 2,5-Dimethylresorcin, 4-Chlorresorcin, 3-Amino-phenol, 2-Amino-3-hydroxypyridin, 2-Chlor-6-methyl-3-aminophenol, 2,6-Dihydroxy-3,4-dimethyl-pyridin, 2-({3-[(2-Hydroxyethyl)amino]-4-methoxy-5-methylphenyl}amino)ethanol, 2-({3-[(2-Hydroxy-ethyl)amino]-2-methoxy-5-methylphenyl}amino)ethanol, 1-Methoxy-2-amino-4-beta-hydroxy-ethyl-aminobenzol (Lehmanns Blau), 2,4-Diaminophenoxyethanol, 5-Amino-4-chloro-o-Kresol, 2,4-Dichlor-m-aminophenol, 2,6-Dihydroxy-3,4-dimethylpyridin und/oder einem physiologisch verträglichen Salz dieser Verbindungen.

Ebenfalls gemäß Erfindung bevorzugte Mittel sind dadurch gekennzeichnet, dass sie die mindestens eine Kupplerkomponente und/oder deren physiologisch verträgliches Salz in einem Gewichtsanteil von 0,001 bis 5,0 Gew.-%, mehr bevorzugt von 0,025 bis 2,5 Gew.-%, besonders bevorzugt von 0,05 bis 2 Gew.-% und insbesondere von 0,1 bis 1,5 Gew.-%, jeweils bezogen auf das Gesamtgewicht des anwendungsbereiten Mittels, enthalten.

Um eine ausgewogene und subtile Nuancenausbildung zu erzielen, ist es gemäß Erfindung vorteilhaft, wenn weitere farbgebende Komponenten im erfindungsgemäßen Mittel enthalten sind.

Es kann daher erfindungsgemäß bevorzugt sein, wenn das erfindungsgemäße bzw. im erfindungsgemäßen Verfahren eingesetzte Mittel mindestens eine weitere farbgebende Komponente enthält, die ausgewählt ist aus zusätzlichen Oxidationsfarbstoffvorprodukten vom Entwicklertyp und/oder direktziehenden Farbstoffen.

Bevorzugte weitere Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)-propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, 4-Aminophenol, 4-Amino-3-methylphenol, 4-Amino-2-aminomethylphenol, 4-Amino-2-(1,2-dihydroxyethyl)-phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Besonders bevorzugte zusätzliche Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]-amin, und/oder 4,5-Diamino-1-(2-hydroxyethyl)-pyrazol sowie deren physiologisch verträglichen Salze.

Die Entwicklerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Zusammenfassend sind erfindungsgemäße bzw. im erfindungsgemäßen Verfahren eingesetzte Färbemittel solche, die, jeweils bezogen auf ihr Gewicht, 0,05 bis 5 Gew.-%, vorzugsweise 0,1 bis 4,5 Gew.-%, weiter bevorzugt 0,15 bis 4 Gew.-%, noch weiter bevorzugt 0,2 bis 3,5 Gew.-% und insbesondere 0,25 bis 3 Gew.-% Oxidationsfarbstoffvorprodukte enthalten.

In Ergänzung zu Oxidationsfarbstoffvorprodukten können die erfindungsgemäßen bzw. im erfindungsgemäßen Verfahren eingesetzten Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 3 Gew.-%.

Direktziehende Farbstoffe können in anionische, kationische und nichtionische direktziehende Farbstoffe unterteilt werden, die der Anforderungen der Trägerbasis vom Fachmann ausgewählt und eingesetzt werden.

Bevorzugte anionische direktziehende Farbstoffe sind die unter den internationalen Bezeichnungen bzw. Handelsnamen Bromphenolblau, Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), Basic Blue 99, Basic Brown 16 und Basic Brown 17 sowie Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Bevorzugte erfindungsgemäße Färbemittel bzw. in erfindungsgemäß bevorzugten Verfahren eingesetzte Färbemittel können auch naturanaloge Farbstoffe enthalten. Erfindungsgemäß bevorzugte Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich vor der Anwendung nicht mit einem zusätzlichen Oxidationsmittel versetzt.

Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Besonders gut als Vorstufen naturanaloger Haarfarbstoffe geeignet sind Derivate des 5,6-Dihydroxyindolins, insbesondere 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure, sowie weiterhin Derivate des 5,6-Dihydroxyindols, insbesondere 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, sowie physiologisch verträgliche Salze der vorstehend genannten Verbindungen.

Zusammenfassend sind erfindungsgemäße bzw. im erfindungsgemäßen Verfahren eingesetzte Färbemittel bevorzugt, die 0,05 bis 5 Gew.-%, vorzugsweise 0,1 bis 4,5 Gew.-%, weiter bevorzugt 0,15 bis 4 Gew.-%, noch weiter bevorzugt 0,2 bis 3,5 Gew.-% und insbesondere 0,25 bis 3 Gew.-% direktziehende(n) Farbstoff(e) enthalten.

Die im gemäß Erfindung bzw. im erfindungsgemäßen Verfahren eingesetzten Mittel enthalten 0,1 bis 5 Gew.-%, jeweils umgerechnet auf die undissoziierte Dicarbonsäure und bezogen auf das Gewicht des Färbemittels, Dicarbonsäure(n) mit 2 bis 10 Kohlenstoffatomen, ausgewählt aus Äpfelsäure, D-Weinsäure, L-Weinsäure, meso-Weinsäure, Traubensäure, alpha-Ketoglutarsäure, beta-Ketoglutarsäure und Oxalessigsäure, und/oder Salz(e) dieser Säure(n), wobei die Dicarbonsäure aus Äpfelsäure ausgewählt ist.

Die genannte Dicarbonsäure leistet einen wesentlichen Beitrag zur verringerten Haarschädigung durch die erfindungsgemäßen Färbemittel.

Die erfindungsgemäßen Färbemittel weisen einen pH-Wert im Bereich von 8,5 bis 11,0, bevorzugt 10 bis 10,5, auf, jeweils gemessen bei 20°C. Je nach pH-Wert des erfindungsgemäßen Färbemittels oder der in einem der erfindungsgemäßen Färbeverfahren eingesetzten Zusammensetzungen kann die mindestens eine Dicarbonsäure mit 2 bis 10 Kohlenstoffatomen als undissoziierte Säure, teilweise dissoziiert oder vollständig dissoziiert vorliegen. Liegt die mindestens eine Dicarbonsäure mit 2 bis 10 Kohlenstoffatomen teilweise dissoziiert oder vollständig dissoziiert vor, so ist das Gegenion ausgewählt aus physiologisch verträglichen Kationen, wie insbesondere den Alkalimetall-, Erdalkalimetall- und Zinkionen sowie Ammoniumionen, Alkylammonium-, Alkanolammonium- und Glucammoniumionen, insbesondere die Mono-, Di- und Trimethyl-, -ethyl- und -hydroxyethylammoniumionen. Ebenfalls bevorzugt sind die Salze der gesättigten Dicarbonsäuren mit 2 bis 10 Kohlenstoffatomen mit Amino-C₁-C₆-Alkanolen, insbesondere mit Monoethanolamin, und Amino-C₁-C₆-Alkandiolen, insbesondere mit 2-Amino-2-methylpropan-1-ol, 2-Amino-2-methyl-propan-1,3-diol, 2-Aminopropan-1-ol, 3-Aminopropan-1-ol, 1-Aminopropan-2-ol (MIPA) und 2-Amino-2-(hydroxymethyl)propan-1,3-diol (TRIS), wobei die Salze mit Monoethanolamin, 2-Amino-2-methylpropan-1-ol und 2-Amino-2-methylpropan-1,3-diol besonders bevorzugt sind. Außerordentlich bevorzugt sind Natrium-, Kalium-, Magnesium-, Ammonium und Monoethanolammonium-lonen als Gegenionen für die teilweise oder vollständig dissoziierten Dicarbonsäuren mit 2 bis 10 Kohlenstoffatomen. Daneben können jedoch auch mit alkalisch reagierenden Aminosäuren, wie beispielsweise Arginin, Lysin, Ornithin und Histidin, neutralisierte Dicarbonsäuren mit 2 bis 10 Kohlenstoffatomen eingesetzt werden.

Die Natrium-, Kalium-, Ammonium-, Monoethanolammonium-, Lysin- sowie Argininsalze sowie deren Mischungen sind bevorzugte Salze der Dicarbonsäuren mit 2 bis 10 Kohlenstoffatomen. Bevorzugte Färbemittel gemäß der vorliegenden Erfindung enthalten die mindestens eine Dicarbonsäure mit 2 bis 10 Kohlenstoffatomen, ausgewählt aus Äpfelsäure, D-Weinsäure, L-Weinsäure, meso-Weinsäure, Traubensäure, alpha-Ketoglutarsäure, beta-Ketoglutarsäure und Oxalessigsäure, oder ein oder mehrere Salze hiervon in einer Gesamtmenge von 0,2 bis 4 Gew.-%, bevorzugt 0,33 bis 3 Gew.-%, besonders bevorzugt 0,5 bis 2 Gew.-%, jeweils umgerechnet auf die undissoziierte Dicarbonsäure und bezogen auf das Gewicht des Färbemittels, wobei zwingend Äpfelsäure enthalten ist.

Auch wenn die Dicarbonsäuren in Salzform vorliegen, beziehen sich die vorstehenden Mengenangaben auf die jeweilige Dicarbonsäure in undissoziierter Form, um die Mengenangabe nicht durch unterschiedliche Molgewichte der Salze zu verfälschen.

Besonders bevorzugt sind erfindungsgemäße bzw. im erfindungsgemäßen Verfahren eingesetzte Färbemittel bevorzugt, die mindestens eine Dicarbonsäure mit 2 bis 10 Kohlenstoffatomen, ausgewählt aus Äpfelsäure, D-Weinsäure, L-Weinsäure, meso-Weinsäure, Traubensäure, alpha-Ketoglutarsäure, beta-Ketoglutarsäure und Oxalessigsäure, in einer Gesamtmenge von 0,2 bis 4 Gew.-%, bevorzugt 0,33 bis 3 Gew.-%, besonders bevorzugt 0,5 bis 2 Gew.-%, enthalten ist, jeweils umgerechnet auf die undissoziierte Dicarbonsäure und bezogen auf das Gewicht des erfindungsgemäßen Färbemittels, enthalten, wobei zwingend Äpfelsäure enthalten ist.

Ganz besonders bevorzugte erfindungsgemäße bzw. im erfindungsgemäßen Verfahren eingesetzte Färbemittel enthalten, jeweils umgerechnet auf die undissoziierte Dicarbonsäure und bezogen auf das Gewicht des Färbemittels, 0,2 bis 4 Gew.-%, bevorzugt 0,33 bis 3 Gew.-%, besonders bevorzugt 0,5 bis 2 Gew.-% Äpfelsäure.

Die erfindungsgemäßen bzw. im erfindungsgemäßen Verfahren eingesetzten Färbemittel enthalten 20 bis 95 Gew.-% Wasser. Bevorzugte Mittel enthalten 30 bis 90 Gew.-%, besonders bevorzugt 40 bis 85 Gew.-%, außerordentlich bevorzugt 45 bis 82,5 Gew.-% und insbesondere 40 bis 80 Gew.-% Wasser, jeweils bezogen auf das Gesamtgewicht des erfindungsgemäßen Färbemittels.

Die erfindungsgemäßen bzw. im erfindungsgemäßen Verfahren eingesetzten Färbemittel enthalten weniger als 0,1 Gew.-% Peroxidverbindung(en). Überraschenderweise sind aus erfindungsgemäßen Mitteln durch Zugabe von Oxidationsmittelzubereitungen ("Entwicklern") hergestellte Haarfarben gegenüber solchen, die aus herkömmlichen Färbecremes, Entwicklern und einer nachträglichen Zumischung der Dicarbonsäure(n) erhalten wurden, bei ansonsten gleichen Einsatzmengen effektiver im Faserschutz.

Es ist bevorzugt, die erfindungsgemäßen bzw. im erfindungsgemäßen Verfahren eingesetzten Färbemittel noch peroxidärmer zu gestalten. Besonders bevorzugte erfindungsgemäße bzw. im erfindungsgemäßen Verfahren eingesetzte Färbemittel enthalten weniger als 0,01 Gew.-%, vorzugsweise weniger als 0,005, besonders bevorzugt weniger als 0,001 Gew.-% Peroxidverbindungen und sind insbesondere frei von Peroxidverbindungen.

Besonders bevorzugte erfindungsgemäße bzw. im erfindungsgemäßen Verfahren eingesetzte Färbemittel enthalten weniger als 0,01 Gew.-%, vorzugsweise weniger als 0,005, besonders bevorzugt weniger als 0,001 Gew.-% Wasserstoffperoxid (berechnet als 100 %-iges H₂O₂). Ganz besonders bevorzugte Mittel sind vollständig frei von Wasserstoffperoxid.

Besonders bevorzugte erfindungsgemäße bzw. im erfindungsgemäßen Verfahren eingesetzte Färbemittel enthalten weniger als 0,01 Gew.-%, vorzugsweise weniger als 0,005, besonders bevorzugt weniger als 0,001 Gew.-% Kalium-, Natrium- und/oder Ammoniumpersulfat. Ganz besonders bevorzugte Mittel sind vollständig frei von Persulfaten.

Die erfindungsgemäßen bzw. im erfindungsgemäßen Verfahren eingesetzten Färbemittel enthalten weiterhin Mischungen aus Arginin und Lysin in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Aminosäure und bezogen auf das Gewicht des Färbemittels, enthält,
wobei mindestens eine Aminosäure aus der Gruppe Arginin, Lysin, Histidin, Asparagin, Glutamin, Cystein, Methionin, Tryptophan sowie Mischungen hiervon in einer Gesamtmenge von 0,05 bis 3 Gew.-%, bevorzugt 0,1 bis 2 Gew.-%, besonders bevorzugt 0,2 bis 1,2 Gew.-%, enthalten ist, jeweils umgerechnet auf die undissoziierte Aminosäure und bezogen auf das Gewicht des Färbemittels, enthalten ist.

Diese Aminosäuren verleihen den erfindungsgemäßen bzw. im erfindungsgemäßen Verfahren eingesetzten Färbemitteln eine nochmals verbesserte Faserschutzwirkung.

Weiterhin wurde überraschend festgestellt, dass die verringerte Haarschädigungswirkung der erfindungsgemäßen bzw. im erfindungsgemäßen Verfahren eingesetzten Färbemittel weiter unterstützt werden kann, wenn mindestens eine Verbindung der allgemeinen Formel (II) enthalten ist.

Bevorzugte erfindungsgemäße bzw. im erfindungsgemäßen Verfahren eingesetzte Färbemittel enthalten daher zusätzlich mindestens eine Verbindung der allgemeinen Formel (II) 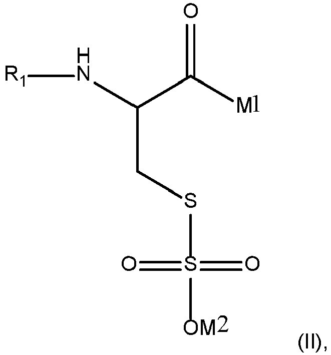 wobei
R1 für ein Wasserstoffatom oder für ein Strukturelement der Formel (IV) steht 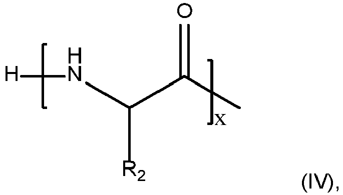 wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-lndol-3-ylmethyl-Gruppe oder eine (Sulfosulfanyl)methyl-Gruppe,
M1 steht für die Gruppierung -OM2 oder für ein Strukturelement der Formel (V) 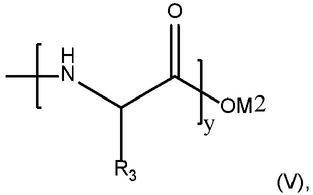
wobei
y für eine ganze Zahl von 1 bis 100 steht,
der Rest R3 in jedem der Strukturelemente der Formel (V) jeweils unabhängig vom vorangegangenen Strukturelement der Formel (V) gewählt werden kann,
R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-lndol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺,
wobei bevorzugt eine oder mehrere Verbindungen der vorstehend aufgeführten Formel (II) in einer Gesamtmenge von 0,001 bis 2,5 Gew.-% weiter bevorzugt von 0,01 bis 1,0 Gew.% und besonders bevorzugt von 0,02 bis 0,1 Gew.-% enthalten sind, jeweils bezogen auf das Gewicht des erfindungsgemäßen Färbemittels.

Bei den optional einzusetzenden Verbindungen der Formel (II) handelt es sich um das Bunte-Salz einer Aminosäure, eines Oligopeptids oder eines Peptids.

Der Rest R1 kann entweder für ein Wasserstoffatom oder für ein Strukturelement der Formel (IV) stehen

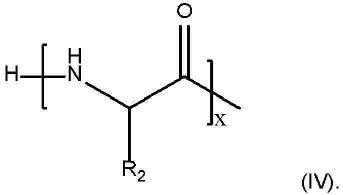

Das Strukturelement der Formel (IV) ist weiterhin gekennzeichnet durch den Wiederholungsindex x, wobei x für eine ganze Zahl von 1 bis 100 steht. Der Wiederholungsindex x gibt an, wie viele Strukturelemente der Formel (IV) in der Verbindung der Formel (II) enthalten sind.

Bevorzugt steht x für eine ganze Zahl von 1 bis 50, weiter bevorzugt steht x für eine ganze Zahl von 1 bis 20, und ganz besonders bevorzugt steht x für eine ganze Zahl von 1 bis 10.

Wenn x beispielweise für die Zahl 10 steht, beinhaltet die Verbindung der Formel (II) 10 Strukturelemente der Formel (IV).

Hierbei ist wesentlich, dass der Rest R2 in jedem der Strukturelemente der Formel (IV) jeweils unabhängig vom vorangegangenen Strukturelement der Formel (IV) gewählt werden kann. Enthalten die Verbindungen der Formel (II) beispielsweise 10 Struktureinheiten der Formel (IV), so können diese 10 Struktureinheiten gleich oder aber auch verschieden sein.

Der Rest R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder für eine (Sulfosulfanyl)methyl-Gruppe.

Bei dem Strukturelement der Formel (IV) handelt es sich somit um eine Aminosäure, die peptidisch über ihre Amino- und/oder ihre Säurefunktion innerhalb der Verbindung der Formel (II) verknüpft ist. Wenn es sich bei der Aminosäure um Cystein handelt, kann diese auch in Form eines Bunte-Salzes vorliegen.

Wenn der Rest R2 für ein Wasserstoffatom steht, so beruht das Strukturelement der Formel (IV) auf der Aminosäure Glycin. Ist R2 eine Methylgruppe, so beruht das Strukturelement der Formel (IV) auf der Aminosäure Alanin.

Analog gilt:

| **Wenn der Rest R2 für ... steht,** | **beruht das Strukturelement der Formel (IV) auf der Aminosäure** |
|---|---|
| (H₃C)₂CH- | Valin |
| (H₃C)₂CH-CH₂- | Leucin |
| H₃C-CH₂-CH(CH₃)- | Isoleucin |
| C₆H₅-CH₂- | Phenylalanin |
| 4-OH-C₆H₅-CH₂- | Tyrosin |
| HO-CH₂- | Serin |
| H₃C-CH(OH)- | Threonin |
| H₂N-CH₂-CH₂-CH₂-CH₂- | Lysin |
| H₂N-C(NH)-NH-CH₂-CH₂-CH₂- | Arginin |
| HOOC-CH₂-CH₂- | Glutaminsäure |
| HOOC-CH₂- | Asparaginsäure |
| H₂N-C(O)-CH₂-CH₂- | Glutamin |
| H₂N-C(O)-CH₂- | Asparagin |
| HS-CH₂- | Cystein |
| H₃C-S-CH₂-CH₂- | Methionin |
| 1H-Imidazol-4-ylmethyl- | Histidin |
| 1H-Indol-3-ylmethyl- | Tryptophan |

Schließlich kann der Rest R2 auch für eine (Sulfosulfanyl)methyl-Gruppe stehen, hierbei handelt es sich um eine Bunte-Salz-Struktur der Formel HO-S(O₂)-S-CH₂-.

Je nach pH-Wert des Färbemittels kann die Bunte-Salz-Struktur der Formel HO-S(O₂)-S-CH₂- auch in ihrer deprotonierten Form vorliegen.

Innerhalb der Verbindung der Formel (II) steht M1 steht die Gruppierung -OM2 oder für ein Strukturelement der Formel (V)

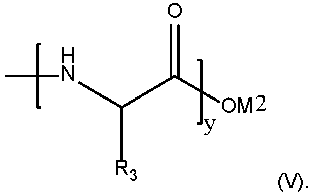

Das Strukturelement der Formel (V) ist gekennzeichnet durch den Wiederholungsindex y, wobei y für eine ganze Zahl von 1 bis 100 steht. Der Wiederholungsindex y gibt an, wie viele Strukturelemente der Formel (V) in der Verbindung der Formel (II) enthalten sind.

Bevorzugt steht y für eine ganze Zahl von 1 bis 50, weiter bevorzugt steht y für eine ganze Zahl von 1 bis 20, und ganz besonders bevorzugt steht y für eine ganze Zahl von 1 bis 10.

Wenn y beispielweise für die Zahl 10 steht, beinhaltet die Verbindung der Formel (II) 10 Strukturelemente der Formel (V).

Hierbei ist wesentlich, dass der Rest R3 in jedem der Strukturelemente der Formel (V) jeweils unabhängig vom vorangegangenen Strukturelement der Formel (V) gewählt werden kann. Enthalten die Verbindungen der Formel (II) beispielsweise 10 Struktureinheiten der Formel (V), so können diese 10 Struktureinheiten gleich oder aber auch verschieden sein.

Der Rest R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methyl-sulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe.

Damit handelt es sich auch bei dem Strukturelement der Formel (V) um eine Aminosäure, die peptidisch über ihre Amino- und/oder ihre Säurefunktion innerhalb der Verbindung der Formel (II) verknüpft ist. Wenn es sich bei der Aminosäure um Cystein handelt, kann diese auch in Form eines Bunte-Salzes vorliegen.

| **Wenn der Rest R3 für** ... **steht,** | **beruht das Strukturelement der Formel (V) auf der Aminosäure** |
|---|---|
| -H | Glycin |
| -CH₃ | Alanin |
| (H₃C)₂CH- | Valin |
| (H₃C)₂CH-CH₂- | Leucin |
| H₃C-CH₂-CH(CH₃)- | Isoleucin |
| C₆H₅-CH₂- | Phenylalanin |
| 4-OH-C₆H₅-CH₂- | Tyrosin |
| HO-CH₂- | Serin |
| H₃C-CH(OH)- | Threonin |
| H₂N-CH₂-CH₂-CH₂-CH₂- | Lysin |
| H₂N-C(NH)-NH-CH₂-CH₂-CH₂- | Arginin |
| HOOC-CH₂-CH₂- | Glutaminsäure |
| HOOC-CH₂- | Asparaginsäure |
| H₂N-C(O)-CH₂-CH₂- | Glutamin |
| H₂N-C(O)-CH₂- | Asparagin |
| HS-CH₂- | Cystein |
| H₃C-S-CH₂-CH₂- | Methionin |
| 1H-Imidazol-4-ylmethyl- | Histidin |
| 1H-Indol-3-ylmethyl- | Tryptophan |

Schließlich kann der Rest R3 auch für eine (Sulfosulfanyl)methyl-Gruppe stehen, hierbei handelt es sich um eine Bunte-Salz Struktur der Formel HO-S(O₂)-S-CH₂-.

Je nach pH-Wert des Färbemittels kann auch hier die Bunte-Salz Struktur der Formel HO-S(O₂)-S-CH₂- auch in ihrer deprotonierten Form vorliegen.

Der Rest M2 steht für ein Wasserstoffatom, ein Äquivalent eines ein oder mehrwertigen Kations oder ein Ammoniumion (NH₄)⁺.

Als bevorzugte Äquivalente eines ein oder mehrwertigen Kations können insbesondere die Kationen von Natrium und Kalium (Na⁺ bzw. K⁺) oder auch Magnesium oder Calcium (1/2 Mg²⁺ oder ½ Ca²⁺) genannt werden.

Steht M2 für ein Wasserstoffatom, so handelt es sich bei der Gruppierung -OM2 um die Gruppierung -OH. Steht M2 für ein Natriumkation, so handelt es sich bei der Gruppierung -OM2 um die Gruppierung -ONa. Steht M2 für ein Kaliumkation, so handelt es sich bei der Gruppierung -OM2 um die Gruppierung -OK. Steht M2 für ein Ammoniumion, so handelt es sich bei der Gruppierung -OM2 um die Gruppierung -O(NH₄).

Die Gruppierung -OM2 ist stets einer Carbonylgruppe benachbart. In Summe liegt, wenn M2 für H, K, Na oder Ammonium steht, damit in der Verbindung der Formel (II) daher entweder in Form eine Säure in ihrer protonierten Form oder aber das Natrium, Kalium oder Ammoniumsalz dieser Säure vor.

Bei den erfindungsgemäßen Verbindungen der Formel (II) handelt es sich entweder um das Bunte-Salz der Aminosäure Cystein, um die Bunte-Salze von Oligo-Peptiden oder aber um die Bunte-Salze von Peptiden.

Wenn der Rest R1 für ein Wasserstoffatom steht und der Rest M1 für eine Gruppierung -OM2 steht, dann handelt es sich bei der Verbindung der Formel (II) um das Bunte-Salz der Aminosäure Cystein. In diesem Fall handelt es sich bei der Verbindung der Formel (II) um die Verbindung der Formel (IIa), 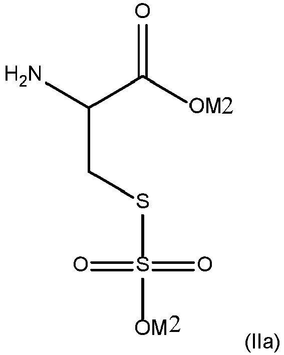 wobei M2 wieder wie zuvor beschrieben definiert.

Liegt die Verbindung der Formel (IIa) in Form ihrer freien Säure vor, so handelt es sich um 2-Amino-3-(sulfosulfanyl)propansäure. Diese Substanz ist kommerziell erhältlich.

Es hat sich herausgestellt, dass der Einsatz der Verbindung der Formel (IIa) in Färbemitteln bereits bei besonders geringen Einsatzmengen zu einer besonders effektiven Reduzierung der Haarschädigung führt, die auch nach wiederholten Haarwäschen noch vorhanden ist. Deshalb ist der Einsatz von Verbindungen der Formel (IIa) ganz besonders bevorzugt.

In einer ganz besonders bevorzugten Ausführungsform ist ein erfindungsgemäßes oder im erfindungsgemäßen Verfahren eingesetztes Färbemittel, dadurch gekennzeichnet, dass es mindestens eine Verbindung der Formel (II) enthält, wobei
R1 für ein Wasserstoffatom steht und
M1 für eine Gruppierung -OM2 steht.

Wenn eine Verbindung der Formel (IIa) eingesetzt wird, so handelt es sich bevorzugt um den Einsatz dieser spezifischen Verbindung. Werden als Verbindungen der Formel (II) jedoch die Bunte-Salze von Oligopeptiden eingesetzt, so kann das erfindungsgemäße Färbemittel auch mehrere Verbindungen der Formel (II) als Gemisch verschiedener Oligopeptide enthalten. Diese Oligopeptide werden durch ihr mittleres Molgewicht definiert. Das mittlere Molekulargewicht M_{w} des mindestens einen Oligopeptids der Formel (II) kann beispielsweise durch Gelpermeationschromatographie (GPC) mit Polystyrol als internem Standard bestimmt werden.

Je nachdem, wie viele Strukturelemente der Formel (IV) und/oder (V) in der Verbindung der Formel (II) enthält sind, und je nach Art dieser Aminosäuren kann das Molgewicht der erfindungsgemäß verwendeten Verbindung der Formel (II) variieren. Es ist erfindungsgemäß besonders bevorzugt, wenn es sich bei der Verbindung der Formel (II) um ein Oligopeptid handelt, das ein Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Unter dem Begriff "Oligopeptid" im Rahmen der vorliegenden Erfindung werden Kondensationsprodukte von Aminosäuren verstanden, welche die oben genannten Molekulargewichte besitzen.

In einer ganz besonders bevorzugten Ausführungsform ist ein erfindungsgemäßes Färbemittel dadurch gekennzeichnet, dass es mindestens eine Verbindung der Formel (II) enthält, die ein Molekulargewicht M_{w} von 200 bis 2.000 Da (Dalton), vorzugsweise von 250 bis 1.500 Da, bevorzugt von 300 bis 1.200 Da, insbesondere von 400 bis 800 Da aufweist.

Wird im erfindungsgemäßen Färbemittel eine Mischung von Oligomeren eingesetzt, so können diese Mischungen durch ihr mittleres Molekulargewicht definiert werden.

In diesem Fall ist ein bevorzugtes erfindungsgemäßes Färbemittel dadurch gekennzeichnet, dass es mindestens eine Mischung von Verbindungen der Formel (II) enthält, die ein mittleres Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Weiterhin hat sich gezeigt, dass der Schutz- oder Repair-Effekt, den die Verbindungen der Formel (II) besitzen, auch von den Wiederholungsindices x und y abhängt. Wie zuvor beschrieben, ist es ganz besonders bevorzugt, wenn x für eine ganze Zahl von 1 bis 10 steht und y für eine ganze Zahl von 1 bis 10 steht.

In einer weiteren ganz besonders bevorzugten Ausführungsform ist ein erfindungsgemäßes Färbeoder Blondiermittel dadurch gekennzeichnet, dass es mindestens eine Verbindung der Formel (II) enthält, wobei
R1 für ein Strukturelement der Formel (IV) steht, und
M1 für ein Strukturelement der Formel (V) steht, und
x für eine ganze Zahl von 1 bis 10 steht und
y für eine ganze Zahl von 1 bis 10 steht.

Neben dem Molekulargewicht der Verbindung der Formel (II) nimmt auch der Anteil der Bunte-Salz Einheiten, die in der Verbindung der Formel (II) enthalten sind, einen entscheidenden Einfluss auf die Wirksamkeit der Schutzwirkung oder "Repair-Wirkung" der Verbindungen.

Verbindungen mit mindestens einer Bunte-Salz-Einheit - wie sie beispielsweise in der Verbindung der Formel (IIa) vorhanden ist - sind sehr effektiv, insbesondere wenn sie als monomere Verbindung eingesetzt werden. Oligopeptide mit mindestens einer Bunte-Salz Einheit sind besonders wirksam, wenn sie ein niedriges Molekulargewicht von bis zu 1200, insbesondere 800 Dalton besitzen.

Bei Einsatz von Oligopeptiden ist es jedoch von ganz besonderem Vorteil, wenn die Verbindung der Formel (II) mindestens zwei, bevorzugt mindestens drei Bunte-Salz Einheiten besitzt.

In einer weiteren ganz besonders bevorzugten Ausführungsform ist ein erfindungsgemäßes Färbemittel dadurch gekennzeichnet, dass es mindestens eine Verbindung der Formel (II) enthält, wobei
R1 für ein Strukturelement der Formel (IV) steht, und
der Rest R2 in mindestens einem Strukturelement der Formel (IV) für eine (Sulfosulfanyl)methyl-Gruppe (d.h. eine Gruppe HO-S(O₂)-S-CH₂-) steht.

In einer weiteren ganz besonders bevorzugten Ausführungsform ist ein erfindungsgemäßes Färbemittel dadurch gekennzeichnet, dass es mindestens eine Verbindung der Formel (II) enthält, wobei
R1 für ein Strukturelement der Formel (IV) steht, und
x für eine ganze Zahl von mindestens 3 steht und
der Rest R2 in mindestens 3 Strukturelementen der Formel (IV) für eine 2-Carboxyethyl-Gruppe (d.h. eine Gruppe HOOC-CH2-CH2-) steht.

In einer weiteren ganz besonders bevorzugten Ausführungsform ist ein erfindungsgemäßes Färbemittel dadurch gekennzeichnet, dass es mindestens eine Verbindung der Formel (II) enthält, wobei
M1 für ein Strukturelement der Formel (V) steht, und
y für eine ganze Zahl von mindestens 3 steht und
der Rest R3 in mindestens 3 Strukturelementen der Formel (IV) für eine Gruppe (Glu) steht.

Die mindestens eine Verbindung der Formel (II) ist - bezogen auf das Gesamtgewicht des erfindungsgemäß bevorzugten Färbemittels in einer Gesamtmenge von 0,001 bis 10 Gew.-% enthalten.

Überraschenderweise hat sich jedoch herausgestellt, dass die Verbindung(en) der Formel (II) bereits in geringen Einsatzkonzentrationen eine sehr gute Reduzierung der Haarschädigung bewirken können. Aus diesem Grund ist es besonders vorteilhaft, wenn das erfindungsgemäß bevorzugte Färbemittel eine oder mehrere Verbindungen der vorstehend aufgeführten Formel (II) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht des erfindungsgemäßen Färbemittels.

In einer weiteren ganz besonders bevorzugten Ausführungsform ist ein erfindungsgemäßes Färbemittel dadurch gekennzeichnet, dass es eine oder mehrere Verbindungen der vorstehend aufgeführten Formel (II) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht des erfindungsgemäßen Färbemittels.

Weiterhin wurde überraschend festgestellt, dass die verringerte Haarschädigungswirkung der erfindungsgemäßen bzw. im erfindungsgemäßen Verfahren eingesetzten Färbemittel weiter unterstützt werden kann, wenn bestimmte Polymere enthalten sind.

Bevorzugte erfindungsgemäße bzw. im erfindungsgemäßen Verfahren eingesetzte Färbemittel enthalten daher zusätzlich mindestens ein Polymer A, das mindestens zehn konstitutive Einheiten der Formel (I) aufweist, 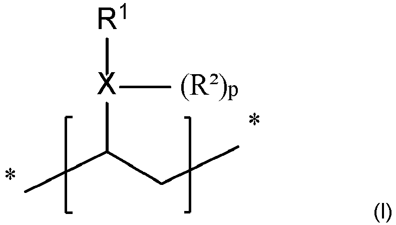 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält,
wobei das mindestens eine Polymer A mit mindestens zehn konstitutiven Einheiten der Formel (I) bevorzugt in einer Gesamtmenge von 0,2 bis 5 Gew.-%, besonders bevorzugt 0,5 bis 3 Gew.-%, außerordentlich bevorzugt 1,0 bis 2,3 Gew.-%, jeweils bezogen auf das Gewicht des Färbemittels, enthalten ist.

Unter "Polymer" werden im Sinne der vorliegenden Anmeldung Polymere im Sinne der IUPAC-Definition verstanden, die mindestens 10 identische konstitutive Einheiten umfassen.

Die Anzahl an konstitutiven Einheiten in einem Polymer bezeichnet man als Polymerisationsgrad. Erfindungsgemäß bevorzugte Polymere A weisen jeweils einen Polymerisationsgrad im Bereich von 40 bis 1000, bevorzugt 100 bis 800, besonders bevorzugt 350 bis 650, auf. Weitere erfindungsgemäß bevorzugte Polymere A mit mindestens zehn konstitutiven Einheit der Formel (I) enthalten 40 bis 1000, bevorzugt 100 bis 800, besonders bevorzugt 350 bis 650 identische konstitutive Einheiten der Formel (I).

R¹ und R² stehen bevorzugt jeweils unabhängig voneinander für Wasserstoff oder für eine C₂-C₁₀-Acylgruppe, die bevorzugt ausgewählt aus einer Acetyl-, Propanoyl-, oder n-Butanoyl-Gruppe, besonders bevorzugt ausgewählt aus einer Acetyl-Gruppe.

Erfindungsgemäß bevorzugte Polymere A weisen mindestens 10 konstitutive Einheiten der Formel (I) auf, in denen X für Stickstoff steht, wobei das Polymer A keine permanent-ionischen konstitutiven Einheiten enthält.

Weitere erfindungsgemäß besonders bevorzugte Polymere A weisen mindestens 10 konstitutive Einheiten der Formel (I) auf, in denen X für Stickstoff steht und R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist.

Wenn R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, so ist dieser Ring bevorzugt mit mindestens einer funktionellen Gruppe substituiert, die ausgewählt ist aus =O. Ein besonders bevorzugte Substituenten-Kombination X, R¹, R² ist eine Pyrrolidon-Gruppe, so dass es sich bei einer erfindungsgemäß besonders bevorzugten konstitutiven Einheit der Formel (I) um eine Einheit der Formel (la) handelt, 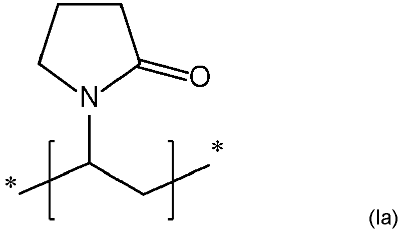 in der X für Stickstoff steht und R¹ und R² zusammen mit diesem Stickstoffatom einen fünfgliedrigen gesättigten Ring bilden, der keine weiteren Heteroatome enthält und der in 2-Position mit einer funktionellen Gruppe =O substituiert ist.

Eine weitere besonders bevorzugte Substituenten-Kombination X, R¹, R² ist eine ε-Caprolactam-Gruppe, so dass es sich bei einer erfindungsgemäß besonders bevorzugten konstitutiven Einheit der Formel (I) um eine Einheit der Formel (I b) handelt,

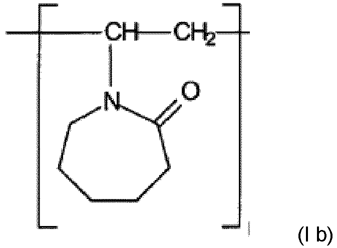

in der X für Stickstoff steht und R¹ und R² zusammen mit diesem Stickstoffatom einen sechsgliedrigen gesättigten Ring bilden, der keine weiteren Heteroatome enthält und der mit einer funktionellen Gruppe =O substituiert ist.

Eine weitere besonders bevorzugte Substituenten-Kombination X, R¹, R² ist eine Imidazol-Gruppe, so dass es sich bei einer weiteren erfindungsgemäß besonders bevorzugten Einheit der Formel (I) um eine Einheit handelt, in der X für Stickstoff steht und R¹ und R² zusammen mit diesem Stickstoffatom einen fünfgliedrigen ungesättigten Ring bilden, der als weiteres Heteroatom Stickstoff enthält.

Weitere erfindungsgemäß bevorzugte Polymere A weisen zu 25 - 100 Mol-%, bevorzugt zu 55 - 100 Mol-%, besonders bevorzugt zu 85 - 100 Mol-% konstitutive Einheiten der Formel (I) auf, in denen X für Stickstoff steht, wobei das Polymer A keine permanent-ionischen konstitutiven Einheiten enthält.

Weitere erfindungsgemäß bevorzugte Polymere A weisen zu 25 - 100 Mol-%, bevorzugt zu 55 - 100 Mol-%, besonders bevorzugt zu 85 - 100 Mol-% konstitutive Einheiten der Formel (I) auf, in denen X für Stickstoff steht und R¹ und R² zusammen mit X einen fünf- oder sechsgliedrigen, gesättigten oder ungesättigten Ring bilden, der gegebenenfalls weitere Heteroatome enthält, die ausgewählt sind aus N und O und gegebenenfalls mit mindestens einer C1 - C6-Alkylgruppe und/oder mit mindestens einer funktionellen Gruppe substituiert ist, wobei das Polymer A keine permanent-ionischen konstitutiven Einheiten enthält.

Erfindungsgemäß besonders bevorzugte Polymere A weisen zu 98 - 100 Mol-% konstitutive Einheiten der Formel (la) auf, wobei das Polymer A keine permanent-ionischen konstitutiven Einheiten enthält.

Erfindungsgemäß außerordentlich bevorzugte Polymere A weisen zu 98 - 100 Mol-% konstitutive Einheiten der Formel (la) auf und haben einen Polymerisationsgrad im Bereich von 40 bis 1000, bevorzugt 100 bis 800, besonders bevorzugt 350 bis 650, wobei das Polymer A keine permanent-ionischen konstitutiven Einheiten enthält. Besonders bevorzugte Polymere A sind Polyvinylpyrrolidon-Homopolymere mit einem Polymerisationsgrad im Bereich von 40 bis 1000, bevorzugt 100 bis 800, besonders bevorzugt 350 bis 650.

Eine weitere besonders bevorzugte Substituenten-Kombination X, R¹, R² ist eine konstitutive Einheit der Formel (I), in der X für Sauerstoff steht, p null ist und R¹ für Wasserstoff steht.

Eine weitere besonders bevorzugte Substituenten-Kombination X, R¹, R² ist eine konstitutive Einheit der Formel (I), in der X für Sauerstoff steht, p null ist und R¹ für eine Acetylgruppe steht.

Weitere erfindungsgemäß bevorzugte Polymere A enthalten zu 75-92 Mol-% konstitutive Einheiten der Formel (I), in der X für Sauerstoff steht, p null ist und R¹ für Wasserstoff steht, und zu 8 - 25 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten 40 bis 1000, bevorzugt 100 bis 800, besonders bevorzugt 350 bis 650 konstitutive Einheiten der Formel (I), davon zu 75-92 Mol-% konstitutive Einheiten der Formel (I), in der X für Sauerstoff steht, p null ist und R¹ für Wasserstoff steht, und zu 8 - 25 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten zu 65-25 Mol-% konstitutive Einheiten der Formel (la) und zu 35 - 75 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten 40 bis 1000, bevorzugt 100 bis 800, besonders bevorzugt 350 bis 650 konstitutive Einheiten der Formel (I), davon zu 65-25 Mol-% konstitutive Einheiten der Formel (la) und zu 35 - 75 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Das mindestens eine Polymer A mit mindestens zehn konstitutiven Einheiten der Formel (I) weist keine permanenten ionischen Ladungen auf. Es ist aber möglich, dass die konstitutiven Einheiten der Formel (I), beispielsweise durch Protonierung des Stickstoffatoms in einem sauren Träger, ionisch, insbesondere kationisch, vorliegen. Diese Ladungen sind aber nicht permanent, sondern temporär, da sie vom umgebenden Medium abhängig sind.

Bevorzugte erfindungsgemäße Färbemittel enthalten das mindestens eine Polymer A mit mindestens zehn konstitutiven Einheiten der Formel (I) in einer Gesamtmenge von 0,2 bis 5 Gew.-%, bevorzugt 0,5 bis 3 Gew.-%, besonders bevorzugt 1,0 bis 2,3 Gew.-%, jeweils bezogen auf das Gewicht des Färbemittels.

Als weiteren optionalen Inhaltsstoff enthalten erfindungsgemäß bevorzugte Färbemittel mindestens ein permanent kationisches Polymer B.

Bevorzugt enthält das permanent kationische Polymer neben mindestens einem permanent kationisch geladenen Monomer-Typ auch mindestens einen permanent anionisch geladenen Monomer-Typ, wobei die kationischen Monomere im molaren Überschuss zu den anionischen Monomeren vorliegen, so dass das mindestens eine zweite erfindungsgemäße Polymer eine kationische Nettoladung aufweist. Derartige erfindungsgemäß bevorzugte Polymere werden auch als amphotere oder zwitterionische Polymere bezeichnet.

In einer ersten bevorzugten Ausführungsform enthalten erfindungsgemäß bevorzugte Färbe- oder Blondiermittel mindestens ein permanent kationisches Polymer, das ausgewählt ist aus
- kationischen Polymeren, die aufgebaut sind aus Monomeren mit quartären Ammoniumgruppen der allgemeinen Formel (a),

   R³-CH=CR⁴-CO-Z-(CₙH₂ₙ)-N⁽⁺⁾R⁵R⁶R⁷ A⁽⁻⁾ (a),

   in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt, bevorzugt ausgewählt aus kationischen Polymeren, die aufgebaut sind aus Acrylamidopropyltrimethylammoniumchlorid,
   besonders bevorzugt ausgewählt aus amphoteren Polymeren mit kationischer Nettoladung, die durch Polymerisation aufgebaut sind aus
      a) kationischen Monomeren mit quartären Ammoniumgruppen der allgemeinen Formel (a),

         R³-CH=CR⁴-CO-Z-(CₙH₂ₙ)-N⁽⁺⁾R⁵R⁶R⁷ A⁽⁻⁾ (a),

         in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt, und
      b) mindestens einer ungesättigten Carbonsäure, ausgewählt aus Acrylsäure, Methacrylsäure und Crotonsäure sowie aus Mischungen dieser Säuren, wobei die mindestens eine ungesättigte Carbonsäure in Form ihrer Salze vorliegen kann,
      wobei im Polymer die kationischen Monomere im molaren Überschuß zu den anionischen Monomeren vorliegen;
   außerordentlich bevorzugt ausgewählt aus amphoteren Polymeren mit kationischer Nettoladung, die den mindestens einen Monomer-Typ der allgemeinen Formel (a) und den mindestens einen Monomer-Typ der ungesättigten Carbonsäure, ausgewählt aus Acrylsäure, Methacrylsäure und Crotonsäure sowie Mischungen hiervon, in einem Molverhältnis von 60:40 bis 95:5, bevorzugt von 75:25 bis 90:10 zueinander enthalten,
   außerordentlich bevorzugt ausgewählt aus amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen;
- 2-[2-Hydroxy-3-(trimethylammonio)propoxy]ethylcelluloseetherchlorid, das beispielsweise unter der INCI-Bezeichnung Polyquaternium-10 erhältlich ist,
- Terpolymeren aus Acrylsäure, Diallyldimethylammoniumchlorid und Acrylamid, wie sie beispielsweise unter der INCI-Bezeichnung Polyquaternium-39 erhältlich sind,
- Homopolymeren des N,N,N-Trimethyl-2-[(methyl-1-oxo-2-propenyl)oxy]ethanaminiumchlorid, wie sie beispielsweise unter der INCI-Bezeichnung Polyquaternium-37 erhältlich sind,
- Copolymeren aus Diallyldimethylammoniumchlorid und Acrylsäure, wie sie beispielsweise unter der INCI-Bezeichnung Polyquaternium-22 erhältlich sind,
- Hydroxyethylcellulose-Dimethyldiallylammoniumchlorid-Copolymeren, wie sie beispielsweise unter der INCI-Bezeichnung Polyquaternium-4 erhältlich sind,
- Copolymeren aus Acrylamid und beta-Methacrylyloxyethyltrimethylammoniummethosulfat, wie sie beispielsweise unter der INCI-Bezeichnung Polyquaternium-5 erhältlich sind,
- Homopolymeren des N,N-Dimethyl-N-2-Propenyl-2-Propen-1-aminiumchlorid, wie sie beispielsweise unter der INCI-Bezeichnung Polyquaternium-6 erhältlich sind,
- Copolymeren aus Diallyldimethylammoniumchlorid und Acrylamid, wie sie beispielsweise unter der INCI-Bezeichnung Polyquaternium-7 erhältlich sind,
- Copolymeren aus Vinylpyrrolidon und Dimethylaminoethylmethacrylatdiethylsulfat, wie sie beispielsweise unter der INCI-Bezeichnung Polyquaternium-11 erhältlich sind,
- sowie Mischungen der vorgenannten Polymere.

Erfindungsgemäß außerordentlich bevorzugte permanente kationische Polymere sind ausgewählt aus 2-[2-Hydroxy-3-(trimethylammonio)propoxy]ethylcelluloseetherchlorid, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen und Terpolymeren aus Acrylsäure, Diallyldimethylammoniumchlorid und Acrylamid, sowie Zweierund Dreier-Mischungen dieser Polymere.

Besonders bevorzugte Polymer B-Mischungen enthalten 2-[2-Hydroxy-3-(trimethylammonio)propoxy]ethylcelluloseetherchlorid und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten 2-[2-Hydroxy-3-(trimethylammonio)propoxy]ethylcelluloseetherchlorid, mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und mindestens ein Terpolymer aus Acrylsäure, Diallyldimethylammoniumchlorid und Acrylamid.

Erfindungsgemäß ebenfalls außerordentlich bevorzugte permanent-kationische Polymere B sind ausgewählt aus Polyquaternium-10, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen, und Polyquaternium-39, sowie Zweierund Dreier-Mischungen dieser Polymere.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und Polyquaternium-39.

Bevorzugte erfindungsgemäße Färbemittel enthalten das mindestens eine permanent kationische Polymer B in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen Färbemittels.

Die erfindungsgemäße bzw. im erfindungsgemäßen Verfahren verwendete Färbezubereitung enthält vorzugsweise mindestens ein Alkalisierungsmittel, ausgewählt aus Ammoniumhydroxid, Monoethanolamin und Natriumsilikaten sowie Mischungen hiervon.

Zur Erzielung der gewünschten haltbaren Färbung der keratinischen Fasern muss ein Färbemittel einen pH-Wert im Bereich von 6,5 bis 11,0, bevorzugt 8 bis 10,5, besonders bevorzugt 8,5 bis 10, jeweils gemessen bei 20°C, aufweisen. Bei diesen pH-Werten öffnet sich die äußere Keratinfaserschicht optimal zur Aufnahme der Oxidationsfarbstoffvorprodukte und entfaltet sich die gewünschte Wirkung der über die Entwickleremulsion zugesetzten Peroxidverbindung optimal.

Die erfindungsgemäßen bzw. im erfindungsgemäßen Verfahren verwendeten Färbezubereitungen weisen einen pH-Wert im Bereich von 8,5 bis 11,0, bevorzugt 10 bis 10,5, jeweils gemessen bei 20°C, auf.

Bevorzugt wird Ammoniak in Form seiner wässrigen Lösung eingesetzt. Bei entsprechenden wässrigen Ammoniak-Lösungen kann es sich um 10 bis 35 prozentige Lösungen handeln (berechnet in Gew.-%, 100 g wässrige Ammoniaklösung enthalten dementsprechend 10 bis 35 g Ammoniak). Bevorzugt wird Ammoniak in Form einer 20 bis 30 Gew.-%igen Lösung, besonders bevorzugt in Form einer 25 Gew.-%igen Lösung eingesetzt.

In einer besonders bevorzugten Ausführungsform ist das erfindungsgemäße Färbemittel dadurch gekennzeichnet, dass es Ammoniumhydroxid in einer Menge von 0,20 bis 2,5 Gew.-%, bevorzugt von 0,5 bis 2,0 Gew.-%, weiter bevorzugt von 1,0 bis 1,5 Gew.-% und besonders bevorzugt von 0,31 bis 0,8 Gew.-% - bezogen auf das Gesamtgewicht des erfindungsgemäßen Färbemittels - enthält.

Zusätzlich zu bzw. anstelle von Ammoniumhydroxid enthalten bevorzugte erfindungsgemäße Färbemittel Monoethanolami.

Zur Erzielung einer maximalen Geruchsabdeckung und zur Optimierung der Echtheitseigenschaften ist Monoethanolamin in einer Gesamtmenge von 0,2 - 6,5 Gew.-%, bevorzugt von 0,5 - 4,0 Gew.-%, weiter bevorzugt von 0,7 bis 2,5 Gew.-% und besonders bevorzugt von 0,8 bis 1,6 Gew.% - bezogen auf das Gesamtgewicht des erfindungsgemäßen Färbemittels - enthalten.

Bei Natriumsilikaten im Sinne der vorliegenden Erfindung handelt es sich um chemische Verbindungen, die sich aus Natriumoxid und Siliciumdioxid zusammengesetzen und die in verschiedenen molaren Verhältnissen (Monosilikat, Metasilikat und Polysilikat) vorkommen können. Ein Beispiel für ein Natriumsilikat ist das Natriumsalz der Orthokieselsäure mit der Summenformel Na₄SiO₄, das auch als Natriumorthosilikat bezeichnet wird.

Weitere Beispiele für geeignete Natriumsilikate sind das Dinatriummetasilikat bzw. Natriummetasilikat mit der Summenformel Na₂SiO₃, das Dinatriumdisilikat mit der Summenformel Na₂Si₂O₅ oder das Dinatriumtrisilikat mit der Summenformel Na₂Si₃O₇.

Silikate in amorpher Form können durch das Zusammenschmelzen von Siliciumdioxid und Alkalioxid in molaren Verhältnissen zwischen 1:1 und 4:1 hergestellt werden. Die so erhaltenen Feststoffe werden bei etwa 150 °C und 5 bar Dampfdruck gelöst, um eine Lösung der Natriumsilikate in Wasser zu erhalten, bei diesen entsprechenden Lösungen handelt es sich um Alkaliwassergläser. Als Alkaliwassergläser werden aus einer Schmelze erstarrte, glasartige (amorphe) Natriumsilikate oder ihre wässrigen Lösungen bezeichnet. Diese nennt man auch Natronwasserglas. Auch Natronwassergläser sind innerhalb dieser Erfindung von der Definition der Natriumsilikate umfasst.

Die molare Zusammensetzung bei Wassergläsern beträgt üblicherweise 2 bis 4 mol SiO₂ auf 1 mol Alkalioxid (Na₂O).

Ein Beispiel für ein bevorzugtes Natriumsilikat ist Natronwasserglas, das in Form seiner wässrigen Lösung vorliegt, einen Na₂O-Gehalt von 7,5 bis 8,8 Gew.-% und einen SiO2 Gehalt von 25,0 bis 28,5 Gew.-% besitzt und das die CAS-Nr. 1344-09-5 (Chemical Abstracts Number) hat.

Weitere erfindungsgemäß bevorzugte Färbemittel enthalten mindestens ein Natriumsilikat in einer Gesamtmenge von 0,1 bis 9 Gew.-%, bevorzugt 0,2 bis 8 Gew.-%, besonders bevorzugt 1 bis 7,5 Gew.-%, jeweils bezogen auf das Gesamtgewicht des erfindungsgemäßen Färbemittels.

Weiterhin können andere Alkalisierungsmittel, wie Kaliumhydroxid (KOH) und Natriumhydroxid (NaOH) enthalten sein, meist in einer Gesamtmenge von 0,05 bis 1,5 Gew.-%, bevorzugt 0,1 bis 0,6 Gew.-%, jeweils bezogen auf das Gesamtgewicht des erfindungsgemäßen Färbemittels.

Die erfindungsgemäße bzw. im erfindungsgemäßen Verfahren verwendete Färbezubereitung enthält optional weitere Hilfs- und Zusatzstoffe. So hat es sich erfindungsgemäß als bevorzugt erwiesen, wenn die Färbezubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Ggf. kann schon ein optional enthaltenes Polymer A oder ein optional enthaltenes Polymer B als Verdickungsmittel fungieren. Die nachstehend beschriebenen verdickenden Polymere können somit ggf. unter die Definition für Polymer A oder Polymer B fallen.

Gemäß einer ersten bevorzugten Ausführungsform handelt es sich bei dem Verdickungsmittel um ein anionisches, synthetisches Polymer. Bevorzugte anionische Gruppen sind die Carboxylat- und die Sulfonatgruppe.

Beispiele für anionische Monomere, aus denen die polymeren anionischen Verdickungsmittel bestehen können, sind Acrylsäure, Methacrylsäure, Crotonsäure, Itaconsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind Maleinsäureanhydrid sowie insbesondere 2-Acrylamido-2-methylpropansulfonsäure und Acrylsäure.

Bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichnen Carbopol^{®} im Handel erhältlich. Ebenfalls bevorzugt ist das Homopolymer der 2-Acrylamido-2-methylpropansulfonsäure, das beispielsweise unter der Bezeichnung Rheothik^{®}11-80 im Handel erhältlich ist. Innerhalb dieser ersten Ausführungsform kann es weiterhin bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Itaconsäuremono- und -diester, Vinylpyrrolidinon, Vinylether und Vinylester. Die anionischen Acrylsäure- und/oder Methacrylsäure-Polymerisate oder -Copolymerisate sind in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten. Bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein bevorzugtes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn^{®} 22 sowie von der Firma National Starch unter den Handelsbezeichnungen Structure^{®} 2001 und Structure^{®} 3001 vertrieben.

Bevorzugte anionische Copolymere sind weiterhin Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxythan, Allylsucrose, Allylpentaerythrit und Methylen-bisacrylamid zum Einsatz kommen. Ein solches Polymer ist in den Handelsprodukten Sepigel^{®}305 und Simulgel^{®} 600 der Firma SEPPIC enthalten. Die Verwendung dieser Verbindungen, die neben der Polymerkomponente eine Kohlenwasserstoffmischung (C₁₃-C₁₄-Isoparaffin beziehungsweise Isohexadecan) und einen nichtionogenen Emulgator (Laureth-7 beziehungsweise Polysorbate-80) enthalten, hat sich im Rahmen der erfindungsgemäßen Lehre als besonders vorteilhaft erwiesen. Auch Polymere aus Maleinsäureanhydrid und Methylvinylether, insbesondere solche mit Vernetzungen, sind bevorzugte Verdickungsmittel. Ein mit 1,9-Decadien vernetztes Maleinsäure-Methylvinylether-Copolymer ist unter der Bezeichnung Stabileze^{®} QM im Handel erhältlich.

Bevorzugt kann das erfindungsgemäße bzw. im erfindungsgemäßen Verfahren verwendete Mittel zusätzlich mindestens ein anionisches Acrylsäure- und/oder Methacrylsäure-Polymerisat oder
- Copolymerisat. Bevorzugte Polymerisate dieser Art sind:
   - Polymerisate z.B. aus wenigstens 10 Gew.-% Acrylsäure-Niedrigalkylester, 25 bis 70 Gew.-% Methacrylsäure und ggf. bis zu 40 Gew.-% eines weiteren Comonomeren,
   - Mischpolymerisate aus 50 bis 75 Gew.-% Ethylacrylat, 25 bis 35 Gew.-% Acrylsäure und 0 bis 25 Gew.-% anderer Comonomeren bekannt. Geeignete Dispersionen dieser Art sind im Handel erhältlich, z.B. unter der Handelsbezeichnung Latekoll^{®} D (BASF).
   - Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Gemäß einer weiteren Ausführungsform handelt es sich bei dem Verdickungsmittel um einen kationisches synthetisches Polymer. Bevorzugte kationische Gruppen sind quartäre Ammoniumgruppen. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (HP-1), 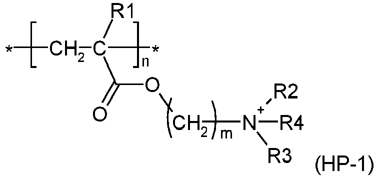 in der R1 = -H oder -CH₃ ist, R2, R3 und R4 unabhängig voneinander ausgewählt sind aus C₁-C₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im Wesentlichen aus den in Formel (HP-1) aufgeführten Monomereinheiten sowie nichtionogenen Monomereinheiten, sind besonders bevorzugte kationische polymere Gelbildner. Im Rahmen dieser Polymeren sind diejenigen erfindungsgemäß bevorzugt, für die mindestens eine der folgenden Bedingungen gilt:
- R1 steht für eine Methylgruppe
- R2, R3 und R4 stehen für Methylgruppen
- m hat den Wert 2,

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

In einer weiteren bevorzugten Ausführungsform werden natürlich vorkommende Verdickungsmittel eingesetzt. Bevorzugte Verdickungsmittel dieser Ausführungsform sind beispielsweise nichtionischen Guargums. Erfindungsgemäß können sowohl modifizierte als auch unmodifizierte Guargums zum Einsatz kommen. Nichtmodifizierte Guargums werden beispielsweise unter der Handelsbezeichnung Jaguar^{®} C von der Firma Rhone Poulenc vertrieben. Erfindungsgemäß bevorzugte modifizierte Guargums enthalten C₁-C₆-Hydroxyalkylgruppen. Bevorzugt sind die Gruppen Hydroxymethyl, Hydroxyethyl, Hydroxypropyl und Hydroxybutyl. Derart modifizierte Guargums sind im Stand der Technik bekannt und können beispielsweise durch Reaktion der Guargums mit Alkylenoxiden hergestellt werden. Der Grad der Hydroxyalkylierung, der der Anzahl der verbrauchten Alkylenoxidmoleküle im Verhältnis zur Zahl der freien Hydroxygruppen der Guargums entspricht, liegt bevorzugt zwischen 0,4 und 1,2. Derart modifizierte Guargums sind unter den Handelsbezeichnungen Jaguar^{®} HP8, Jaguar^{®} HP60, Jaguar^{®} HP120, Jaguar^{®} DC 293 und Jaguar^{®} HP105 der Firma Rhone Poulenc im Handel erhältlich.

Weiterhin geeignete natürliche Verdickungsmittel sind ebenfalls bereits aus dem Stand der Technik bekannt.

Gemäß dieser Ausführungsform bevorzugt sind weiterhin Biosaccharidgums mikrobiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Bevorzugte Hydroxyalkylcellulosen sind insbesondere die Hydroxyethylcellulosen, die unter den Bezeichnungen Cellosize^{®} der Firma Amerchol und Natrosol^{®} der Firma Hercules vertrieben werden. Geeignete Carboxyalkylcellulosen sind insbesondere die Carboxymethylcellulosen, wie sie unter den Bezeichnungen Blanose^{®} von der Firma Aqualon, Aquasorb^{®} und Ambergum^{®} von der Firma Hercules und Cellgon^{®} von der Firma Montello vertrieben werden.

Bevorzugt sind weiterhin Stärke und deren Derivate. Stärke ist ein Speicherstoff von Pflanzen, der vor allem in Knollen und Wurzeln, in Getreide-Samen und in Früchten vorkommt und aus einer Vielzahl von Pflanzen in hoher Ausbeute gewonnen werden kann. Das Polysaccharid, das in kaltem Wasser unlöslich ist und in siedendem Wasser eine kolloidale Lösung bildet, kann beispielsweise aus Kartoffeln, Maniok, Bataten, Maranta, Mais, Getreide, Reis, Hülsenfrüchte wie beispielsweise Erbsen und Bohnen, Bananen oder dem Mark bestimmter Palmensorten (beispielsweise der Sagopalme) gewonnen werden. Erfindungsgemäß einsetzbar sind natürliche, aus Pflanzen gewonnene Stärken und/oder chemisch oder physikalisch modifizierte Stärken. Eine Modifizierung lässt sich beispielsweise durch Einführung unterschiedlicher funktioneller Gruppen an einer oder mehreren der Hydroxylgruppen der Stärke erreichen. Üblicherweise handelt es sich um Ester, Ether oder Amide der Stärke mit gegebenenfalls substituierten C₁-C₄₀-Resten. Besonders vorteilhaft ist eine mit einer 2-Hydroxypropylgruppe veretherte Maisstärke, wie sie beispielsweise von der Firma National Starch unter der Handelsbezeichnung Amaze^{®} vertrieben wird.

Als anorganische Verdickungsmittel haben sich Schichtsilikate (polymere, kristalline Natriumdisilicate) als besonders geeignet im Sinne der vorliegenden Erfindung erwiesen. Insbesondere Tone, insbesondere Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate, wie beispielsweise das von der Firma Süd Chemie unter der Handelsbezeichnung Optigel^{®} vertriebene Magnesiumschichtsilikat, sind bevorzugt.

Zur weiteren Steigerung der Leistung können der erfindungsgemäßen bzw. im erfindungsgemäßen Verfahren verwendeten Zusammensetzung zusätzlich gegebenenfalls hydratisierte SiO₂-Verbindung zugesetzt werden. Es kann erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.% bis 5 Gew.-%, jeweils bezogen auf die Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wässriger Lösung vorliegen.

Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt. Bevorzugt sind Metasilikate, in denen das Verhältnis zwischen n und der Summe aus m und p bei 1:2 oder darunter liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Firma Henkel unter den Bezeichnungen Ferrosil^{®} 119, Natronwasserglas 40/42, Portil^{®} A, Portil^{®} AW und Portil^{®} W und von der Firma Akzo unter der Bezeichnung Britesil^{®} C20 vertrieben.

Vorzugsweise wird den erfindungsgemäßen bzw. im erfindungsgemäßen Verfahren verwendeten Färbemitteln weiterhin ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel RO(CH₂CH₂O)ₓCH₂COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare α-Olefinsulfonate mit 8 bis 24 C-Atomen,
- Sulfonate ungesättigter Fettsäuren mit 8 bis 24 C-Atomen und 1 bis 6 Doppelbindungen,
- α-Sulfofettsäuremethylester von Fettsäuren mit 8 bis 30 C-Atomen,
- Alkylsulfate und Alkylethersulfate der Formel RO(CH₂CH₂O)ₓSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel 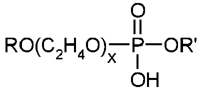 in der R bevorzugt für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht,
- sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht,
   - Monoglyceridsulfate und Monoglyceridethersulfate.

Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonatoder Sulfat-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das C₁₂-C₁₈-Acylsarcosin.

Weiterhin hat es sich als vorteilhaft erwiesen, wenn die erfindungsgemäßen Färbe- und Aufhellmittel weitere, nichtionogene grenzflächenaktive Stoffe, enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 1 bis 50 Mol Ethylenoxid und/oder 0 bis 5 Mol Propylenoxid an lineare und verzweigte Fettalkohole mit 8 bis 30 C-Atomen, wie beispielsweise beispielsweise Lauryl-, Myristyl-, Cetyl-, aber auch Stearyl-, Isostearyl- und Oleylalkohol, an Fettsäuren mit 8 bis 30 C-Atomen und an Alkylphenole mit 8 bis 15 C-Atomen in der Alkylgruppe,
- mit einem Methyl- oder C₂-C₆-Alkylrest endgruppenverschlossene Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- Polyglycerinester und alkoxylierte Polyglycerinester, wie beispielsweise Poly(3)glycerin-diisostearat (Handelsprodukt: Lameform^{®}TGI (Henkel)) und Poly(2)glycerinpolyhydroxy-stearat (Handelsprodukt: Dehymuls^{®}PGPH (Henkel)).
- Polyolfettsäureester, wie beispielsweise das Handelsprodukt Hydagen^{®} HSP (Cognis) oder Sovermol-Typen (Cognis),
- höher alkoxylierte, bevorzugt propoxylierte und insbesondere ethoxylierte, Mono-, Di- und Triglyceride, wie beispielsweise Glycerinmonolaurat + 20 Ethylenoxid und Glycerinmonostearat + 20 Ethylenoxid,
- Aminoxide,
- Hydroxymischether,
- Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO),
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylphenole und Alkylphenolalkoxylate mit 6 bis 21, insbesondere 6 bis 15 Kohlenstoffatomen in der Alkylkette und 1 bis 30 Ethylenoxid- und/oder Propylenoxid-Einheiten. Bevorzugte Vertreter dieser Klasse sind beispielsweise Nonylphenol + 9 EO und Octylphenol + 8 EO;
- Alkylpolyglykoside entsprechend der allgemeinen Formel RO-(Z)ₓ, wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor. Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Die anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar.

Die Alkylamidoamine werden üblicherweise durch Amidierung natürlicher oder synthetischer Fettsäuren und Fettsäureschnitte mit Dialkylaminoaminen hergestellt und zeichnen sich neben einer guten konditionierenden Wirkung speziell durch ihre gute biologische Abbaubarkeit aus. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar. Ebenfalls sehr gut biologisch abbaubar sind quaternäre Esterverbindungen, sogenannte "Esterquats". Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehy-quart^{®} C-4046, Dehyquart^{®} L80 und Dehyquart^{®} AU-35 sind Beispiele für solche Esterquats.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.% sind besonders bevorzugt.

In einer bevorzugten Ausführungsform können nicht-ionische, zwitterionische und/oder amphotere Tenside sowie deren Mischungen bevorzugt sein.

In einer weiteren bevorzugten Ausführungsform kann die Wirkung erfindungsgemäßer bzw im erfindungsgemäßen Verfahren verwendeter Färbemittel durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3 bis 6 Kohlenstoffatomen mit gesättigten Fettsäuren mit 8 bis 22 C-Atomen,
- Sterine, wobei als Sterine eine Gruppe von Steroiden verstanden wird, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide, vor allem Glucose-Phospolipide, die z. B. als Lecithine bzw. Phosphatidylcholine aus z.B. Eidotter oder Pflanzensamen (z.B. Sojabohnen) gewonnen werden,
- Fettsäureester von Zuckern und Zuckeralkoholen, wie Sorbit
- Polyglycerine und Polyglycerinderivate wie beispielsweise Polyglycerinpoly-12-hydroxystearat (Handelsprodukt Dehymuls^{®} PGPH)
- Lineare und verzweigte Fettsäuren mit 8 bis 30 C-Atomen und deren Na-, K-, Ammonium-, Ca, Mg- und Zn-Salze.

Die erfindungsgemäßen bzw im erfindungsgemäßen Verfahren verwendeten Mittel enthalten die Emulgatoren bevorzugt in Mengen von 0,1 bis 25 Gew.-%, insbesondere 0,5 bis 15 Gew.-%, bezogen auf die Gesamtmenge des anwendungsbereiten Mittel.

Nichtionogene Emulgatoren bzw. Tenside mit einem HLB-Wert von 10-15 können erfindungsgemäß besonders bevorzugt sein. Unter den genannten Emulgatoren-Typen können die Emulgatoren, welche kein Ethylenoxid und/oder Propylenoxid im Molekül enthalten ganz besonders bevorzugt sein.

Die Auswahl dieser weiteren Stoffe wird der Fachmann gemäß der gewünschten Eigenschaften der Mittel treffen.

Das erfindungsgemäße Oxidationsfärbemittel wird bei dem erfindungsgemäßen Färbeverfahren mit einer Oxidationsmittelzubereitung (B) zu einem anwendungsbereiten Mittel vermischt, die mindestens eine Peroxidverbindung, die bevorzugt Wasserstoffperoxid ist, enthält, wobei die Zusammensetzung (B) bevorzugt einen pH-Wert im Bereich von 2,5 bis 6,5, bevorzugt 3,0 - 5,5, besonders bevorzugt 3,5 bis 5,0, aufweist, jeweils gemessen bei 20°C.

Bevorzugte Oxidationsmittel sind ausgewählt aus Peroxoverbindungen, bevorzugt ausgewählt aus Wasserstoffperoxid, festen Anlagerungsverbindung von Wasserstoffperoxid an anorganische oder organische Verbindungen, wie Natriumperborat, Natriumpercarbonat, Magnesiumpercarbonat, Natriumpercarbamid, Polyvinylpyrrolidon n H₂O₂ (n ist eine positive ganze Zahl größer 0), Harnstoffperoxid und Melaminperoxid, weiterhin ausgewählt aus Diammoniumperoxodisulfat (auch als Ammoniumpersulfat bezeichnet), Dinatriumperoxodisulfat (auch als Natriumpersulfat bezeichnet) und Dikaliumperoxodisulfat (auch als Kaliumpersulfat bezeichnet) sowie aus Mischungen dieser Oxidationsmittel. Erfindungsgemäß ganz besonders bevorzugt verwendete Oxidationsmittel sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; bevorzugt werden 6- bis 12-Gewichtsprozentige Lösungen in Wasser verwendet. Erfindungsgemäß bevorzugte Oxidationsfärbemittel sind dadurch gekennzeichnet, dass die zu ihrer Herstellung eingesetzte Zusammensetzung (B) - bezogen auf ihr Gewicht - 1 - 24 Gew.-%, bevorzugt 4 - 10 Gew.-%, besonders bevorzugt 3 - 6 Gew.-% Wasserstoffperoxid (berechnet als 100%iges H₂O₂) enthält.

Für oxidative Haarfärbeverfahren wird üblicherweise kurz vor der Applikation auf das Haar das erfindungsgemäße Färbemittel, das ein oder mehrere Oxidationsfarbstoffvorprodukte und ggf. ein oder mehrere direktziehende Farbstoffe enthält, mit einer wässrigen Oxidationsmittel-haltigen Zusammensetzung (B) zu dem anwendungsbereiten Mittel vermischt und dann auf das Haar appliziert. Meistens sind das erfindungsgemäße Färbemittel (A) und die Oxidationsmittel-haltige Zusammensetzung (B) so aufeinander abgestimmt, dass bei einem Mischungsverhältnis von 1 zu 1, bezogen auf Gewichtsteile, im Haarfärbemittel eine Anfangskonzentration an Wasserstoffperoxid von 0,5 - 12 Gew.-%, bevorzugt 2 - 10 Gew.-%, besonders bevorzugt 3 - 6 Gew.-% Wasserstoffperoxid (berechnet als 100%iges H₂O₂) bezogen auf das Gewicht des Oxidationsfärbemittel vorliegt. Es ist aber genauso gut möglich, das erfindungsgemäße Färbemittel (A) und die Oxidationsmittel-haltige Zusammensetzung (B) so aufeinander abzustimmen, dass sich die im anwendungsbereiten Oxidationsfärbemittel notwendigen Konzentrationen durch andere Mischungsverhältnisse als 1:1 ergeben, beispielsweise durch ein Gewichts-bezogenes Mischungsverhältnis von 1:2 oder 1:3 oder auch 2:3.

Für eine Färbung, die eine starke Aufhellung sehr dunklen Haares erfordert, ist der Einsatz von Wasserstoffperoxid oder dessen Anlagerungsprodukten an organische bzw. anorganische Verbindungen oftmals nicht ausreichend. In diesen Fällen wird in der Regel eine Kombination aus Wasserstoffperoxid und Peroxodisulfatsalzen (Persulfatsalzen) eingesetzt. Bevorzugte Persulfatsalze sind Ammoniumperoxidisulfat, Kaliumperoxidisulfat, Natriumperoxidisulfat, sowie Mischungen hiervon.

Das mindestens eine Persulfatsalz ist bevorzugt in einer Gesamtmenge von 0,1 bis 25 Gew.-%, besonders bevorzugt in einer Gesamtmenge von 1 bis 15 Gew.-%, bezogen auf das Gewicht des erfindungsgemäßen Oxidationsfärbemittels, enthalten.

### Beispiele:

Es wurden folgende Färbecremes hergestellt (Angaben in Gew.-%):

| **Zusammensetzung** | **E1** | **E2** | **E3** | **E4** |
|---|---|---|---|---|
| Water, demineralized | ad 100 | | | |
| Propanediol-1,2 | 6,0 | 6,0 | 6,0 | 6,0 |
| Cetearyl Alcohol | 9,0 | 9,0 | 9,0 | 9,0 |
| Ceteareth-20 | 2,4 | 2,4 | 2,4 | 2,4 |
| Sterareth-100 | 0,6 | 0,6 | 0,6 | 0,6 |
| Paraffinum Liquidum | 2,5 | 2,5 | 2,5 | 2,5 |
| Glyceryl Monostearate | 0,5 | 0,5 | 0,5 | 0,5 |
| Sodium sulfite anhydrous | 0,1 | 0,1 | 0,1 | 0,1 |
| EDETA Powder | 0,5 | 0,5 | 0,5 | 0,5 |
| Hydrolyzed Wheat protein | 0,2 | 0,2 | 0,2 | 0,2 |
| Hydrolyzed silk protein | 0,2 | 0,2 | 0,2 | 0,2 |
| PVP solution 30 % | - | 2,0 | 2,0 | 2,0 |
| L-Arginine | 0,2 | 0,2 | 0,2 | 0,2 |
| Lysine HCl | 0,2 | 0,2 | 0,2 | 0,2 |
| Äpfelsäure | 1,5 | 1,5 | 1,5 | 1,5 |
| D-/L-Weinsäure (racemisch) | - | - | - | 0,2 |
| Vitamin C e 300 DAB | - | 0,05 | 0,05 | 0,05 |
| DC CE-8411 Smooth Plus Emulsion | - | 2,0 | 2,0 | 2,0 |
| Apricot kernel oil | - | 0,2 | 0,2 | 0,2 |
| Ammonia 25 % | 12,0 | 12,0 | 12,0 | 12,0 |
| Parfum | 0,5 | 0,5 | 0,5 | 0,5 |
| p-Toluylenediamine sulfate | 0,2 | 0,2 | 0,2 | 0,2 |
| Resorcinol | 0,08 | 0,08 | 0,1 | 0,1 |
| 2-Methylresorcinol | 0,020 | 0,020 | - | |
| 4-Chlororesorcinol | 0,02 | 0,02 | - | |
| 2-Amino-3-hydroxypyridine | 0,009 | 0,009 | - | |
| m-Aminophenol | 0,004 | 0,004 | 0,01 | 0,01 |
| 4-Amino-3-Methylphenol | - | - | 0,02 | 0,02 |
| p-Amino-o-cresol | - | - | 0,008 | 0,008 |

Beispiel 6 ist nicht erfindungsgemäß.

| **Zusammensetzung** | **E5** | **6** | **E7** | **E8** |
|---|---|---|---|---|
| Water, demineralized | ad 100 | | | |
| Carbomer | 0,1 | 0,1 | 0,1 | 0,1 |
| Ammonia 25 % | 12,5 | 12,5 | 12,5 | 12,5 |
| Fatty Alcohol Sulfate-Na C16-18 | 0,8 | 0,8 | 0,8 | 0,8 |
| Sodium Laureth Sulfate 27% | 5,1 | 5,1 | 5,1 | 5,1 |
| Potassium hydroxide 50% | 1,2 | 1,2 | 1,2 | 1,2 |
| Oleic Acid | 0,4 | 0,4 | 0,4 | 0,4 |
| EDETA Powder | 0,2 | 0,2 | 0,2 | 0,2 |
| Glyceryl Monostearate | 4,6 | 4,6 | 4,6 | 4,62 |
| 2-Octyldodecanol | 2,3 | 2,3 | 2,3 | 2,3 |
| Cetearyl Alcohol | 13,9 | 13,9 | 13,9 | 13,9 |
| Ceteareth-20 | 3,5 | 3,5 | 3,5 | 3,5 |
| Sodium sulfite anhydrous | 0,1 | 0,1 | 0,1 | 0,1 |
| PVP solution 30 % | 1,5 | 1,5 | - | - |
| L-Arginine | 0,2 | 0,2 | 0,2 | 0,2 |
| Lysine HCl | 0,2 | 0,2 | 0,2 | 0,2 |
| D-/L-Weinsäure (racemisch) | - | 1,0 | - | - |
| Kaliumtartrat (D-/L-Racemat) | - | - | 1,0 | - |
| Äpfelsäure | 1,0 | - | 1,0 | 2,0 |
| Vitamin C e 300 DAB | 0,05 | 0,05 | 0,05 | 0,05 |
| Parfum | 0,4 | 0,4 | 0,4 | 0,4 |
| p-Toluylenediamine sulfate | 0,1 | 0,1 | 0,1 | 0,1 |
| Resorcinol | 0,06 | 0,06 | 0,06 | 0,06 |
| 2-Methylresorcinol | 0,003 | 0,003 | 0,003 | 0,003 |
| p-Amino-o-cresol | 0,002 | 0,002 | 0,002 | 0,002 |
| 4-Amino-3-nitrophenol | 0,01 | 0,01 | 0,01 | 0,01 |

## Patentansprüche

1. Färbemittel für keratinische Fasern, insbesondere für Humanhaar, enthaltend - bezogen auf ihr Gewicht -
a) mindestens eine Verbindung, ausgewählt aus der Gruppe von Oxidationsfarbstoffvorprodukten, direktziehenden Farbstoffen sowie deren Mischungen,
b) 0,1 bis 5 Gew.-%, jeweils umgerechnet auf die undissoziierte Dicarbonsäure und bezogen auf das Gewicht des Färbemittels, Dicarbonsäure(n) mit 2 bis 10 Kohlenstoffatomen, ausgewählt aus Äpfelsäure, D-Weinsäure, L-Weinsäure, meso-Weinsäure, Traubensäure, alpha-Ketoglutarsäure, beta-Ketoglutarsäure und Oxalessigsäure, und/oder Salz(e) dieser Säure(n),
c) 20 bis 95 Gew.-% Wasser und
d) weniger als 0,1 Gew.-% Peroxidverbindung(en),
**dadurch gekennzeichnet, dass** 0,05 bis 5 Gew.-%, vorzugsweise 0,1 bis 4,5 Gew.-%, weiter bevorzugt 0,15 bis 4 Gew.-%, noch weiter bevorzugt 0,2 bis 3,5 Gew.-% und insbesondere 0,25 bis 3 Gew.-% Oxidationsfarbstoffvorprodukte enthalten sind,
weiterhin **dadurch gekennzeichnet, dass** die mindestens eine Dicarbonsäure mit 2 bis 10 Kohlenstoffatomen ausgewählt ist aus Äpfelsäure,
weiterhin **dadurch gekennzeichnet, dass** es zusätzlich Mischungen aus Arginin und Lysin, in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Aminosäure und bezogen auf das Gewicht des Färbemittels, enthält, wobei
mindestens eine Aminosäure aus der Gruppe Arginin, Lysin, Histidin, Asparagin, Glutamin, Cystein, Methionin, Tryptophan sowie Mischungen hiervon in einer Gesamtmenge von 0,05 bis 3 Gew.-%, bevorzugt 0,1 bis 2 Gew.-%, besonders bevorzugt 0,2 bis 1,2 Gew.-%, enthalten ist, jeweils umgerechnet auf die undissoziierte Aminosäure und bezogen auf das Gewicht des Färbemittels, und
**gekennzeichnet durch** einen pH-Wert im Bereich von 8,5 bis 11,0, bevorzugt 10 bis 10,5, jeweils gemessen bei 20°C.

2. Färbemittel nach Anspruch 1, **dadurch gekennzeichnet, dass** es 0,05 bis 5 Gew.-%, vorzugsweise 0,1 bis 4,5 Gew.-%, weiter bevorzugt 0,15 bis 4 Gew.-%, noch weiter bevorzugt 0,2 bis 3,5 Gew.-% und insbesondere 0,25 bis 3 Gew.-% direktziehende(n) Farbstoff(e) enthält.

3. Färbemittel nach einem der Ansprüche 1 oder 2, **dadurch gekennzeichnet, dass** die mindestens eine Dicarbonsäure mit 2 bis 10 Kohlenstoffatomen in einer Gesamtmenge von 0,2 bis 4 Gew.-%, bevorzugt 0,33 bis 3 Gew.-%, besonders bevorzugt 0,5 bis 2 Gew.-%, enthalten ist, jeweils umgerechnet auf die undissoziierte Dicarbonsäure und bezogen auf das Gewicht des Färbemittels.

4. Färbemittel nach einem der Ansprüche 1 bis 3, **dadurch gekennzeichnet, dass** es weniger als 0,01 Gew.-%, vorzugsweise weniger als 0,005, besonders bevorzugt weniger als 0,001 Gew.% Peroxidverbindungen enthält und insbesondere frei ist von Peroxidverbindungen.

5. Färbemittel nach einem der Ansprüche 1 bis 4, **dadurch gekennzeichnet, dass** es zusätzlich mindestens eine Verbindung der allgemeinen Formel (III) enthält 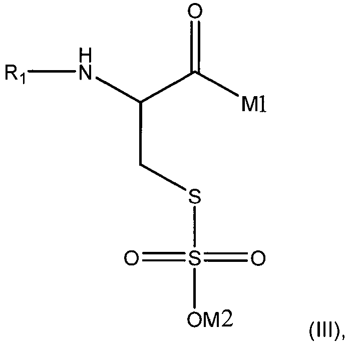 wobei
R1 für ein Wasserstoffatom oder für ein Strukturelement der Formel (IV) steht 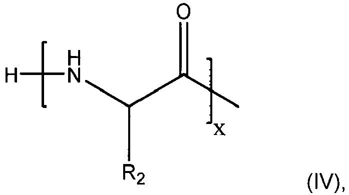 wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-lndol-3-ylmethyl-Gruppe oder eine (Sulfosulfanyl)methyl-Gruppe,
M1 steht für die Gruppierung -OM2 oder für ein Strukturelement der Formel (V) 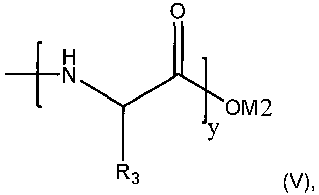
wobei
y für eine ganze Zahl von 1 bis 100 steht,
der Rest R3 in jedem der Strukturelemente der Formel (V) jeweils unabhängig vom vorangegangenen Strukturelement der Formel (V) gewählt werden kann,
R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺,
wobei bevorzugt eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthalten sind, jeweils bezogen auf das Gewicht des erfindungsgemäßen Färbemittels.

6. Färbemittel nach einem der Ansprüche 1 bis 5, **dadurch gekennzeichnet, dass** es zusätzlich mindestens ein Polymer A enthält, das mindestens zehn konstitutive Einheiten der Formel (I) aufweist, 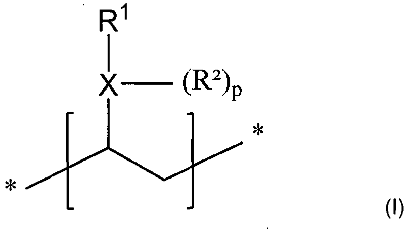 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält,
wobei das mindestens eine Polymer A mit mindestens zehn konstitutiven Einheiten der Formel (I) bevorzugt in einer Gesamtmenge von 0,2 bis 5 Gew.-%, besonders bevorzugt 0,5 bis 3 Gew.-%, außerordentlich bevorzugt 1,0 bis 2,3 Gew.-%, jeweils bezogen auf das Gewicht des Färbemittels, enthalten ist.

7. Verfahren zur oxidativen Färbung keratinischer Fasern, insbesondere von Humanhaar, das folgende Verfahrensschritte umfasst
I. Bereitstellen einer Zusammensetzung (A), enthaltend
a) mindestens eine Verbindung, ausgewählt aus der Gruppe von Oxidationsfarbstoffvorprodukten, direktziehenden Farbstoffen sowie deren Mischungen,
b) 0,1 bis 5 Gew.-%, jeweils umgerechnet auf die undissoziierte Dicarbonsäure und bezogen auf das Gewicht der Zusammensetzung (A), Dicarbonsäure(n) mit 2 bis 10 Kohlenstoffatomen, ausgewählt aus Äpfelsäure, D-Weinsäure, L-Weinsäure, meso-Weinsäure, Traubensäure, alpha-Ketoglutarsäure, beta-Ketoglutarsäure, Oxalessigsäure, Maleinsäure und Fumarsäure, und/oder Salz(e) dieser Säure(n),
c) 20 bis 95 Gew.-% Wasser und
d) weniger als 0,1 Gew.-% Peroxidverbindung(en),
e) 0,05 bis 5 Gew.-%, vorzugsweise 0,1 bis 4,5 Gew.-%, weiter bevorzugt 0,15 bis 4 Gew.-%, noch weiter bevorzugt 0,2 bis 3,5 Gew.-% und insbesondere 0,25 bis 3 Gew.-% Oxidationsfarbstoffvorprodukte,
f) Mischungen aus Arginin und Lysin in einer Gesamtmenge von 0,05 bis 3 Gew.%, bevorzugt 0,1 bis 2 Gew.-%, besonders bevorzugt 0,2 bis 1,2 Gew.-%, jeweils umgerechnet auf die undissoziierte Aminosäure und bezogen auf das Gewicht des Färbemittels, wobei
mindestens eine Aminosäure aus der Gruppe Arginin, Lysin, Histidin, Asparagin, Glutamin, Cystein, Methionin, Tryptophan sowie Mischungen hiervon in einer Gesamtmenge von 0,05 bis 3 Gew.-%, bevorzugt 0,1 bis 2 Gew.-%, besonders bevorzugt 0,2 bis 1,2 Gew.-%, enthalten ist, jeweils umgerechnet auf die undissoziierte Aminosäure und bezogen auf das Gewicht des Färbemittels,
**dadurch gekennzeichnet, dass** die mindestens eine Dicarbonsäure mit 2 bis 10 Kohlenstoffatomen ausgewählt ist aus Äpfelsäure und
**gekennzeichnet durch** einen pH-Wert im Bereich von 8,5 bis 11,0, bevorzugt 10 bis 10,5, jeweils gemessen bei 20°C,
II. Bereitstellen einer Zusammensetzung (B), enthaltend mindestens eine Peroxidverbindung, die bevorzugt Wasserstoffperoxid ist, wobei die Zusammensetzung (B) bevorzugt einen pH-Wert im Bereich von 2,5 bis 6,5, bevorzugt 3,0 - 5,5, besonders bevorzugt 3,5 bis 5,0, aufweist, jeweils gemessen bei 20°C,
III. Vermischen der Zusammensetzungen (A) und (B) miteinander, direkt anschließend
IV. Auftragen der Mischung aus (A) und (B) auf die keratinischen Fasern, insbesondere auf das Humanhaar, und
V. Ausspülen nach einer Einwirkzeit von 0,1 bis 60 Minuten, bevorzugt 1 bis 45 Minuten, besonders bevorzugt 10 bis 30 Minuten,
VI. ggf. weitere Haarbehandlungen, wie Verformen, Konditionieren und/oder Trocknen.

8. Verfahren nach Anspruch 7, **dadurch gekennzeichnet, dass** die Zusammensetzung (A) ein Färbemittel gemäß einem der Ansprüche 1 - 6 ist.

## Claims

1. A colorant for keratin fibers, in particular for human hair, containing, based on its weight,
a) at least one compound selected from the group of oxidation dye precursors, substantive dyes and mixtures thereof,
b) 0.1 to 5 wt.%, in each case converted to the undissociated dicarboxylic acid and based on the weight of the colorant, dicarboxylic acid(s) having 2 to 10 carbon atoms, selected from malic acid, D-tartaric acid, L-tartaric acid, meso-tartaric acid, racemic acid, alpha-ketoglutaric acid, beta-ketoglutaric acid and oxaloacetic acid, and/or salt(s) of these acid(s),
c) 20 to 95 wt.% water, and
d) less than 0.1 wt.% peroxide compound(s),
**characterized in that** 0.05 to 5 wt.%, preferably 0.1 to 4.5 wt.%, more preferably 0.15 to 4 wt.%, even more preferably 0.2 to 3.5 wt.% and in particular 0.25 to 3 wt.% oxidation dye precursors are contained,
further **characterized in that** the at least one dicarboxylic acid having 2 to 10 carbon atoms is selected from malic acid,
further **characterized in that** said colorant also contains mixtures of arginine and lysine in a total amount of 0.05 to 3 wt.%, preferably 0.1 to 2 wt.%, particularly preferably 0.2 to 1.2 wt.%, in each case converted to the undissociated amino acid and based on the weight of the colorant,
at least one amino acid from the group arginine, lysine, histidine, asparagine, glutamine, cysteine, methionine, tryptophan and mixtures thereof being contained in a total amount of 0.05 to 3 wt.%, preferably 0.1 to 2 wt.%, particularly preferably 0.2 to 1.2 wt.%, in each case converted to the undissociated acid and based on the weight of the colorant, and **characterized by** a pH in the range of 8.5 to 11.0, preferably 10 to 10.5, in each case measured at 20 °C.

2. The colorant according to claim 1, **characterized in that** it contains 0.05 to 5 wt.%, preferably 0.1 to 4.5 wt.%, more preferably 0.15 to 4 wt.%, even more preferably 0.2 to 3.5 wt.% and in particular 0.25 to 3 wt.% substantive dye(s).

3. The colorant according to one of claims 1 to 2, **characterized in that** the at least one dicarboxylic acid having 2 to 10 carbon atoms is contained in a total amount of 0.2 to 4 wt.%, preferably 0.33 to 3 wt.%, particularly preferably 0.5 to 2 wt.%, in each case converted to the undissociated dicarboxylic acid and based on the weight of the colorant.

4. The colorant according to one of claims 1 to 3, **characterized in that** it contains less than 0.01 wt.%, preferably less than 0.005, particularly preferably less than 0.001 wt.% peroxide compounds and in particular is free of peroxide compounds.

5. The colorant according to one of claims 1 to 4, **characterized in that** it additionally contains at least one compound of general formula (III) 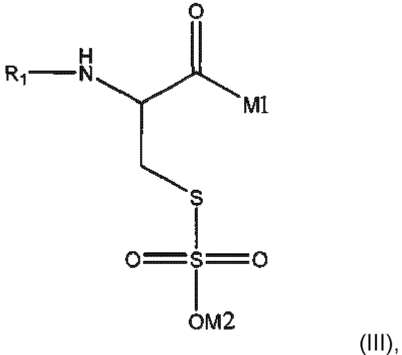 where
R1 represents a hydrogen atom or a structural element of formula (IV) 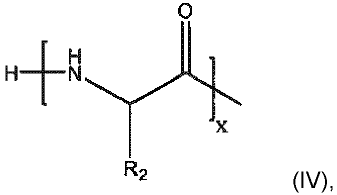 where
x represents an integer from 1 to 100,
the functional group R2 in each of the structural elements of formula (IV) can in each case be selected independently of the preceding structural element of formula (IV), R2 represents a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1H-imidazol-4-ylmethyl group, a 1 H-indol-3-ylmethyl group or a (sulfosulfanyl)methyl group,
M1 represents the group -OM2 or a structural element of formula (V) 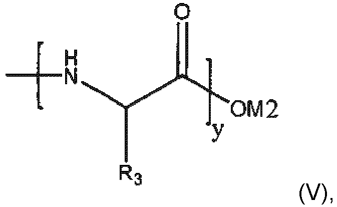
where
y represents an integer from 1 to 100,
the functional group R3 in each of the structural elements of formula (V) can in each case be selected independently of the preceding structural element of formula (V), R3 represents a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1H-imidazol-4-ylmethyl group, a 1 H-indol-3-ylmethyl group or a (sulfosulfanyl)methyl group,
M2 represents a hydrogen atom, an equivalent of a monovalent or polyvalent cation or an ammonium ion (NH₄)⁺,
preferably one or more compounds of the above formula (III) being contained in a total amount of 0.001 to 2.5 wt.%, more preferably 0.01 to 1.0 wt.% and particularly preferably 0.02 to 0.1 wt.%, in each case based on the weight of the colorant according to the invention.

6. The colorant according to one of claims 1 to 5, **characterized in that** it additionally contains at least one polymer A, which has at least ten constituent units of formula (I) 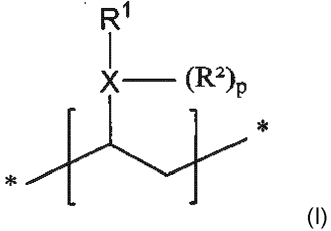 where
- X represents nitrogen or oxygen and
- R¹ and R² each, independently of one another, represent hydrogen or a C2-C10 acyl group, or R¹ and R² together with X form a five-membered or six-membered, saturated or unsaturated ring which optionally contains further heteroatoms preferably selected from N and O and/or is optionally substituted with at least one C1-C6 alkyl group and/or with at least one functional group, and
- p = 0 when X represents oxygen and p = 1 when X represents nitrogen, polymer A containing no permanently ionic constituent units, the at least one polymer A having at least ten constituent units of formula (I) preferably being contained in a total amount of 0.2 to 5 wt.%, particularly preferably 0.5 to 3 wt.%, extremely preferably 1.0 to 2.3 wt.%, in each case based on the weight of the colorant.

7. A method for oxidatively dyeing keratin fibers, in particular human hair, which method comprises the following method steps
I. providing a composition (A) containing
a) at least one compound selected from the group of oxidation dye precursors, substantive dyes and mixtures thereof,
b) 0.1 to 5 wt.%, in each case converted to the undissociated dicarboxylic acid and based on the weight of composition (A), dicarboxylic acid(s) having 2 to 10 carbon atoms, selected from malic acid, D-tartaric acid, L-tartaric acid, meso-tartaric acid, racemic acid, alpha-ketoglutaric acid, beta-ketoglutaric acid, oxaloacetic acid, maleic acid and fumaric acid, and/or salt(s) of this/these acid(s),
c) 20 to 95 wt.% water, and
d) less than 0.1 wt.% peroxide compound(s),
e) 0.05 to 5 wt.%, preferably 0.1 to 4.5 wt.%, more preferably 0.15 to 4 wt.%, even more preferably 0.2 to 3.5 wt.% and in particular 0.25 to 3 wt.% oxidation dye precursors,
f) mixtures of arginine and lysine in a total amount of 0.05 to 3 wt.%, preferably 0.1 to 2 wt.%, particularly preferably 0.2 to 1.2 wt.%, in each case converted to the undissociated amino acid and based on the weight of the colorant, at least one amino acid from the group arginine, lysine, histidine, asparagine, glutamine, cysteine, methionine, tryptophan and mixtures thereof being contained in a total amount of 0.05 to 3 wt.%, preferably 0.1 to 2 wt.%, particularly preferably 0.2 to 1.2 wt.%, in each case converted to the undissociated acid and based on the weight of the colorant,
**characterized in that** the at least one dicarboxylic acid having 2 to 10 carbon atoms is selected from malic acid and
**characterized by** a pH in the range of 8.5 to 11.0, preferably 10 to 10.5, in each case measured at 20 °C,
II. providing a composition (B) containing at least one peroxide compound which is preferably hydrogen peroxide, composition (B) preferably having a pH in the range of 2.5 to 6.5, preferably 3.0 to 5.5, particularly preferably 3.5 to 5.0, in each case measured at 20 °C,
III. mixing compositions (A) and (B) with one another, then immediately
IV. applying the mixture of (A) and (B) to the keratin fibers, in particular to the human hair, and
V. rinsing out after a leave-in time of 0.1 to 60 minutes, preferably 1 to 45 minutes, particularly preferably 10 to 30 minutes,
VI. optional further hair treatments, such as styling, conditioning and/or drying.

8. The method according to claim 7, **characterized in that** composition (A) is a colorant according to one of claims 1 to 6.

## Revendications

1. Produit de coloration pour fibres kératiniques, en particulier pour cheveux humains, contenant, par rapport à son poids,
a) au moins un composé choisi dans le groupe des précurseurs de colorant d'oxydation, des colorants à action directe ainsi que de leurs mélanges,
b) 0,1 à 5 % en poids, respectivement convertis en acide dicarboxylique non dissocié et par rapport au poids du produit de coloration, d'un ou de plusieurs acides dicarboxyliques comportant 2 à 10 atomes de carbone, choisis parmi l'acide malique, l'acide D-tartrique, l'acide L-tartrique, l'acide méso-tartrique, l'acide racémique, l'acide alpha-cétoglutarique, l'acide bêta-cétoglutarique et l'acide oxaloacétique, et/ou leurs sels,
c) 20 à 95 % en poids d'eau et
d) moins de 0,1 % en poids d'un ou de plusieurs composés peroxydes,
**caractérisé en ce qu'**il contient 0,05 à 5 % en poids, de préférence 0,1 à 4,5 % en poids, plus préférablement 0,15 à 4 % en poids, de manière davantage préférée 0,2 à 3,5 % en poids et en particulier 0,25 à 3 % en poids de précurseurs de colorant d'oxydation, **caractérisé en outre en ce que** l'au moins un acide dicarboxylique comportant 2 à 10 atomes de carbone est choisi parmi l'acide malique,
**caractérisé en outre en ce qu'**il contient en outre des mélanges d'arginine et de lysine, en une quantité totale de 0,05 à 3 % en poids, de préférence de 0,1 à 2 % en poids, de manière particulièrement préférée de 0,2 à 1,2 % en poids, respectivement convertis en acide aminé non dissocié et par rapport au poids du produit de coloration,
au moins un acide aminé dans le groupe de l'arginine, de la lysine, de l'histidine, de l'asparagine, de la glutamine, de la cystéine, de la méthionine, du tryptophane et de leurs mélanges étant contenu en une quantité totale de 0,05 à 3 % en poids, de préférence de 0,1 à 2 % en poids, de manière particulièrement préférée de 0,2 à 1,2 % en poids, respectivement converti en acide aminé non dissocié et par rapport au poids du produit de coloration, et
**caractérisé par** un pH compris dans la plage de 8,5 à 11,0, de préférence de 10 à 10,5, respectivement mesuré à 20 °C.

2. Produit de coloration selon la revendication 1, **caractérisé en ce qu'**il contient 0,05 à 5 % en poids, de préférence 0,1 à 4,5 % en poids, plus préférablement 0,15 à 4 % en poids, de manière davantage préférée 0,2 à 3,5 % en poids et en particulier 0,25 à 3 % en poids d'un ou de plusieurs colorants à action directe.

3. Produit de coloration selon l'une des revendications 1 à 2, **caractérisé en ce que** l'au moins un acide dicarboxylique comportant 2 à 10 atomes de carbone est contenu en une quantité totale de 0,2 à 4 % en poids, de préférence de 0,33 à 3 % en poids, de manière particulièrement préférée de 0,5 à 2 % en poids, respectivement converti en acide dicarboxylique non dissocié et par rapport au poids du produit de coloration.

4. Produit de coloration selon l'une des revendications 1 à 3, **caractérisé en ce qu'**il contient moins de 0,01 % en poids, de préférence moins de 0,005, de manière particulièrement préférée moins de 0,001 % en poids de composés peroxydes et en particulier est exempt de composés peroxydes.

5. Produit de coloration selon l'une des revendications 1 à 4, **caractérisé en ce qu'**il contient également au moins un composé de formule générale (III) 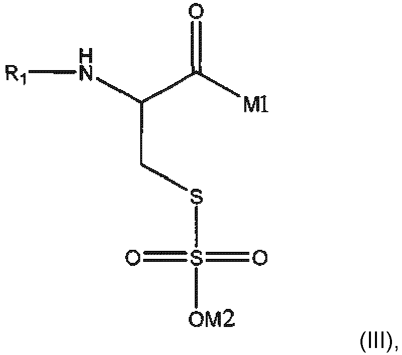 où
R1 représente un atome d'hydrogène ou un élément structurel de formule (IV) 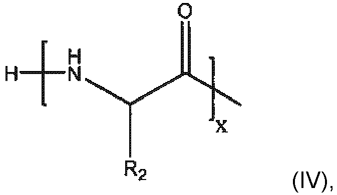 où
x représente un nombre entier compris entre 1 et 100,
le radical R2 dans chacun des éléments structurels de formule (IV) peut être respectivement choisi indépendamment de l'élément structurel précédent de formule (IV),
R2 représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1 H-indol-3-ylméthyle ou un groupe (sulfosulfanyl)méthyle,
M1 représente le groupement -OM2 ou un élément structurel de formule (V) 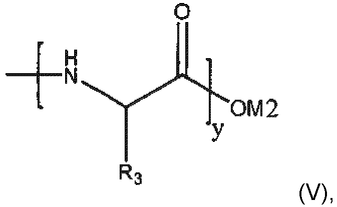
où
y représente un nombre entier compris entre 1 et 100,
le radical R3 dans chacun des éléments structurels de formule (V) peut être respectivement choisi indépendamment de l'élément structurel précédent de formule (V),
R3 représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl)méthyle,
M2 représente un atome d'hydrogène, un équivalent d'un cation monovalent ou polyvalent ou un ion ammonium (NH₄)⁺,
dans lequel de préférence un ou plusieurs composés de formule (III) précédemment mentionnée sont contenus en une quantité totale de 0,001 à 2,5 % en poids, plus préférablement de 0,01 à 1,0 % en poids et de manière particulièrement préférée de 0,02 à 0,1 % en poids, respectivement par rapport au poids du produit de coloration selon l'invention.

6. Produit de coloration selon l'une des revendications 1 à 5, **caractérisé en ce qu'**il contient en outre au moins un polymère A qui présente au moins dix motifs constitutifs de formule (I), 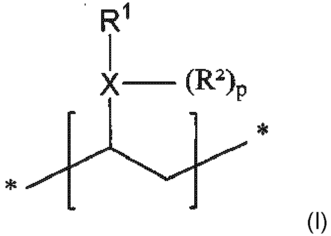 où
- X représente l'azote ou l'oxygène, et
- R¹ et R² représentent, respectivement indépendamment l'un de l'autre, l'hydrogène ou un groupe acyle en C2-C10, ou R¹ et R² forment, conjointement avec X, un cycle à cinq ou six chaînons, saturé ou insaturé, lequel cycle contient éventuellement d'autres hétéroatomes qui sont de préférence choisis parmi N et O, et/ou est éventuellement substitué par au moins un groupe alkyle en C1-C6 et/ou par au moins un groupe fonctionnel, et
- p = 0 si X représente l'oxygène et p = 1 si X représente l'azote, dans lequel le polymère A ne contient pas de motifs constitutifs ioniques permanents, dans lequel l'au moins un polymère A comportant au moins dix motifs constitutifs de formule (I) est contenu de préférence en une quantité totale de 0,2 à 5 % en poids, de manière particulièrement préférée de 0,5 à 3 % en poids, de manière préférée entre toutes de 1,0 à 2,3 % en poids, respectivement par rapport au poids du produit de coloration.

7. Procédé permettant la coloration par oxydation de fibres kératiniques, en particulier de cheveux humains, comprenant les étapes de procédé suivantes
I. préparation d'une composition (A) contenant
a) au moins un composé choisi dans le groupe des précurseurs de colorant d'oxydation, des colorants à action directe ainsi que de leurs mélanges,
b) 0,1 à 5 % en poids, respectivement convertis en acide dicarboxylique non dissocié et par rapport au poids de la composition (A), d'un ou de plusieurs acides dicarboxyliques comportant 2 à 10 atomes de carbone choisis parmi l'acide malique, l'acide D-tartrique, l'acide L-tartrique, l'acide méso-tartrique, l'acide racémique, l'acide alpha-cétoglutarique, l'acide bêta-cétoglutarique, l'acide oxaloacétique, l'acide maléique et l'acide fumarique, et/ou leurs sels,
c) 20 à 95 % en poids d'eau et
d) moins de 0,1 % en poids d'un ou de plusieurs composés peroxydes,
e) 0,05 à 5 % en poids, de préférence 0,1 à 4,5 % en poids, plus préférablement 0,15 à 4 % en poids, de manière davantage préférée 0,2 à 3,5 % en poids et en particulier 0,25 à 3 % en poids, de précurseurs de colorant d'oxydation,
f) des mélanges d'arginine et de lysine en une quantité totale de 0,05 à 3 % en poids, de préférence de 0,1 à 2 % en poids, de manière particulièrement préférée de 0,2 à 1,2 % en poids, respectivement convertis en acide aminé non dissocié et par rapport au poids du produit de coloration,
au moins un acide aminé du groupe de l'arginine, de la lysine, de l'histidine, de l'asparagine, de la glutamine, de la cystéine, de la méthionine, du tryptophane et de leurs mélanges étant contenu en une quantité totale de 0,05 à 3 % en poids, de préférence de 0,1 à 2 % en poids, de manière particulièrement préférée de 0,2 à 1,2 % en poids, respectivement converti en acide aminé non dissocié et par rapport au poids du produit de coloration,
**caractérisé en ce que** l'au moins un acide dicarboxylique comportant 2 à 10 atomes de carbone est choisi parmi l'acide malique et
**caractérisé par** un pH compris dans la plage de 8,5 à 11,0, de préférence de 10 à 10,5, respectivement mesuré à 20 °C,
II. fourniture d'une composition (B) contenant au moins un composé peroxyde qui est de préférence du peroxyde d'hydrogène, la composition (B) présentant de préférence un pH dans la plage de 2,5 à 6,5, de préférence de 3,0 à 5,5, de manière particulièrement préférée de 3,5 à 5,0, respectivement mesuré à 20 °C,
III. mélange des compositions (A) et (B) entre elles, suivi immédiatement par
IV. application du mélange de (A) et (B) sur les fibres kératiniques, en particulier sur les cheveux humains, et
V. rinçage après une durée d'action de 0,1 à 60 minutes, de préférence de 1 à 45 minutes, de manière particulièrement préférée de 10 à 30 minutes,
VI. éventuellement traitements capillaires supplémentaires tels que la mise en forme, le conditionnement et/ou le séchage.

8. Procédé selon la revendication 7, **caractérisé en ce que** la composition (A) est un produit de coloration selon l'une des revendications 1 à 6.
